# EUROPEAN PATENT APPLICATION

(11) **EP 4 706 651 A2**
(43) Date of publication of application: **11.03.2026**
(21) Application number: 25226244.9
(22) Date of filing: 10.05.2022
(51) Int. Cl.: A61K 31/4184

(54) **NEW FORMULATIONS AND USES**

(30) Priority: 10.05.2021 SE 2150595
(62) Divisional of application: 22728545.9
(71) Applicant: AlzeCure Pharma AB, 141 57 Huddinge (SE)
(72) Inventor: HALLDIN, Magnus, 752 26 Uppsala (SE); HAGLUND, Johanna, 129 42 Hägersten (SE); LASSEN, Bo, 15136 Södertälje (SE); SJÖSTRÖM, Eva Maria, 15136 Södertälje (SE); GRIPENHALL, Annika, 15136 Södertälje (SE); SCHIPPER, Nicolaas, 15136 Södertälje (SE); SEGERDAHL, Märta, 141 57 Huddinge (SE)
(74) Representative: Potter Clarkson

(57) **Abstract**

There is provided a pharmaceutical composition comprising the compound N-[(1S)-1-(4-tert-butylphenyl)ethyl]-2-(6,7-difluoro-1H-benzimidazol-1-yl)acetamide, or pharmaceutically acceptable salt thereof, for use in the treatment of pain by topical administration of the composition to a body surface.

## Description

### Field of the Invention

The invention relates to topical compositions and their use in the treatment of pain.

### Prior Art and Background

The listing or discussion of an apparently prior-published document in this specification should not necessarily be taken as an acknowledgement that the document is part of the state of the art or is common general knowledge.

The pain sensation may have a number of underlying causes, principally damage, or the risk of damage, to tissue or nerves. Nociceptive pain is caused by the activation of sensory nerve fibers as a result of harmful stimuli (such as heat or cold) or tissue damage due to trauma or inflammation and neuropathic pain refers to pain caused by damage or disease affecting the nervous system. Neuropathic pain is divided into central and peripheral neuropathic pain, depending on the location of the damaged or diseased nerves. Pain can also be experienced without evidence of disease or damage to tissue or the nervous system. Such pain is referred to as nociplastic pain or sensory hypersensitivity.

Pain may be acute or chronic (persistent pain occurring for an extended period despite medication and/or treatment). Chronic pain is a debilitating condition that is often inadequately treated leading to a poor quality of life and neuropathic pain in particular is often poorly treated. Therefore, there is a significant need for effective treatments to be developed.

The transient receptor potential cation channel subchannel subfamily V member 1 (TrpV1), also referred to as the capsaicin receptor and the vanilloid receptor 1, is mainly located in the neurons of the peripheral nervous system but is also found in many other tissues including in the central nervous system. The TRPV1 receptor is involved in the transmission and modulation of pain signals and is also involved in the detection and regulation of body temperature. The receptor has been the focus of much research into the development of new pain medications, including projects focusing on both agonists (e.g. capsaicin) and antagonists of the receptor.

The oral use of TRPV1 antagonists as potential pain medications has been widely explored, but thus far no products based on this mechanism have been approved.

TRPV1 antagonists including N-[(1S)-1-(4-tert-butylphenyl)ethyl]-2-(6,7-difluoro-1H-benzimidazol-1-yl)acetamide are disclosed in WO 2007/073303 and the use of this compound (later identified by the developmental drug code AZD1386) as an oral analgesic for the pain associated with osteoarthritis and surgical tooth extraction was later explored in clinical trials. However, the development of the compound as an oral medication was terminated due to elevations in liver enzymes being observed (Quiding et al. PAIN, 154 (2013) 808-812).

Topical use of TRPV1 antagonists for the treatment of various types of pain, including particularly, wounds and tissue injuries, is described in WO 2018/048779 and studies showing that the salivary peptide opiorphin and AMG9810 reduced capsaicin-induced wound licking in mice are described. Topical use of TRPV1 antagonists has also been suggested for the treatment of hot flushes (WO 2019/010293).

It has now been found that topically administered N-[(1S)-1-(4-tert-butylphenyl)ethyl]-2-(6,7-difluoro-1H-benzimidazol-1-yl)acetamide is surprisingly effective in the treatment of pain and it has been shown to have a potent and long-lasting analgesic effect when administered topically to the skin.

### Detailed description of the invention

In a first aspect of the invention, there is provided a pharmaceutical composition comprising N-[(1S)-1-(4-tert-butylphenyl)ethyl]-2-(6,7-difluoro-1H-benzimidazol-1-yl)acetamide, 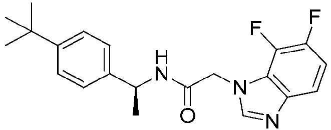 or a pharmaceutically acceptable salt thereof, for use in the treatment of pain, wherein the treatment comprises topical administration of the composition to a body surface.

In an alternative first aspect of the invention, there is provided a method of treating pain comprising topically administering a therapeutically effective amount of pharmaceutical composition comprising the compound N-[(1S)-1-(4-tert-butylphenyl)ethyl]-2-(6,7-difluoro-1H-benzimidazol-1-yl)acetamide, or a pharmaceutically acceptable salt thereof, to a body surface of a patient in need of such treatment.

In a further alternative first aspect of the invention, there is provided the use of a pharmaceutical composition comprising the compound N-[(1S)-1-(4-tert-butylphenyl)ethyl]-2-(6,7-difluoro-1H-benzimidazol-1-yl)acetamide, or a pharmaceutically acceptable salt thereof, for the manufacture of a medicament for the treatment of pain by topical administration of the composition to a body surface.

In a further aspect of the invention, there is provided a pharmaceutical composition comprising the compound N-[(1S)-1-(4-tert-butylphenyl)ethyl]-2-(6,7-difluoro-1H-benzimidazol-1-yl)acetamide, or a pharmaceutically acceptable salt thereof, wherein the composition is formulated for topical use.

The compositions and compositions for use in accordance with the invention (including compositions used in the methods of treatment and the compositions used in the manufacture of medicaments described herein) (hereinafter 'the compositions of the invention') are suitable for, adapted for, and/or packaged and presented for, topical administration. In particular, 'formulated for topical administration' may be understood to indicate that the composition is packaged or presented for topical administration. The compositions may also be understood to comprise the compound N-[(1S)-1-(4-tert-butylphenyl)ethyl]-2-(6,7-difluoro-1H-benzimidazol-1-yl)acetamide, or a pharmaceutically acceptable salt thereof, in admixture with one or more pharmaceutically acceptable topical excipients (such as adjuvants, diluents and/or carriers) (such as the particular excipients described hereinafter).

Preferences, options and particular embodiments described for a given aspect, feature or parameter of the invention should, unless the context indicates otherwise, be regarded as having been disclosed in combination with any and all preferences and options for all other aspects, features and parameters of the invention. In particular, preferences and particular embodiments relating to compositions comprising N-[(1S)-1-(4-tert-butylphenyl)ethyl]-2-(6,7-difluoro-1H-benzimidazol-1-yl)acetamide, or a pharmaceutically acceptable salt thereof, may be understood to apply to the compositions per se as well as to the compositions to be used in medical treatment in accordance with the first aspect of the invention.

Pharmaceutically acceptable salts include acid addition salts and base addition salts. Such salts may be formed by conventional means, for example by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared using techniques known to those skilled in the art, such as by exchanging a counter-ion of a compound of the invention in the form of a salt with another counter-ion, for example using a suitable ion exchange resin.

Particular acid addition salts that may be mentioned include those formed by reaction with corresponding acids, thus protonating the compound of the invention, to form carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxy-benzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulphonate salts (e.g. benzenesulphonate, methyl-, bromo- or chloro-benzenesulphonate, xylenesulphonate, methanesulphonate, ethanesulphonate, propanesulphonate, hydroxy-ethanesulphonate, 1- or 2-naphthalene-sulphonate or 1,5-naphthalene-disulphonate salts) or sulphate, pyrosulphate, bisulphate, sulphite, bisulphite, phosphate, monohydrogenphosphate, dihydrogenphosphate, metaphosphate, pyrophosphate or nitrate salts, and the like.

More particular salts that may be mentioned include hydrogen chloride and, particularly, hydrogen sulfate salts.

In particular embodiments, the compound N-[(1S)-1-(4-tert-butylphenyl)ethyl]-2-(6,7-difluoro-1H-benzimidazol-1-yl)acetamide is added to and/or present in the compositions in free (non-salt) form.

Pharmaceutically acceptable excipients that may be used in the compositions of the invention includes vehicles, adjuvants, carriers, diluents, pH adjusting and buffering agents, tonicity adjusting agents, stabilizers, wetting agents and the like. In particular, such excipients may include adjuvants, diluents or carriers.

In particular embodiments, the compositions of the invention contain the compound N-[(1S)-1-(4-tert-butylphenyl)ethyl]-2-(6,7-difluoro-1H-benzimidazol-1-yl)acetamide in an amount of from about 0.05% (w/w) to about 10% (w/w) (calculated as the free (non salt) compound). In more particular embodiments, the compound N-[(1S)-1-(4-tert-butylphenyl)ethyl]-2-(6,7-difluoro-1H-benzimidazol-1-yl)acetamide is present in an amount of from about 0.05% (w/w) to about 7% (w/w), such as from about 0.05% (w/w) to about 5% (w/w), for example from about 0.5% (w/w) to about 3% (w/w), or from about 0.5% (w/w) to about 3% (w/w) or from about 0.5% (w/w) to about 2.5% (w/w) (e.g. from about 0.5% (w/w) to about 2% (w/w) or from about (0.5% w/w to about 1.5% (w/w)). In further embodiments, the compound N-[(1S)-1-(4-tert-butylphenyl)ethyl]-2-(6,7-difluoro-1H-benzimidazol-1-yl)acetamide is present in an amount of from about 0.05% (w/w) to about 2% (w/w), such as from about 1% (w/w) to about 2% (w/w).

When used herein in relation to a specific value (such as an amount, a period of time or a percentage), the term 'about' (or similar terms, such as 'approximately') may be understood as indicating that such values may vary by up to 10% (particularly, up to 5%, such as up to 1%) of the value defined. It is contemplated that, at each instance, such terms may be replaced with the notation '±10%', or the like (or by indicating a variance of a specific amount calculated based on the relevant value). It is also contemplated that, at each instance, such terms may be deleted.

In particular embodiments, the compositions of the invention further comprise a penetration enhancer component. The penetration enhancer component may be understood to indicate a component that penetrates and/or otherwise interacts with the body surface to promotes flux of the active ingredient into the body surface to which it is applied (e.g. skin). Particular penetration enhancers that may be mentioned include 2-(2-ethoxyethoxy)ethanol (also known as diethylene glycol monoethyl ether, Transcutol^{®}) dimethyl isosorbide, glycerol, ethanol and combinations thereof. More particularly, the penetration enhancer component is selected from 2-(2-ethoxyethoxy)ethanol, dimethyl isosorbide and mixtures thereof. Yet more particularly, the penetration enhancer component is 2-(2-ethoxyethoxy)ethanol.

The penetration enhancer component may be present in the compositions in an amount of from about 10% (w/w) to about 50% (w/w). More particularly, the penetration enhancer component is present in an amount of from about 10% (w/w) to about 40% (w/w), such as from about 20% (w/w) to about 40% (w/w) (e.g. about 25% (w/w) to about 35% (w/w). In particular embodiments, the penetration enhancer component (e.g. 2-(2-ethoxyethoxy)ethanol) is present in an amount of 30% (w/w).

The compositions of the invention may also further comprise a solubility enhancer component. In particular, embodiments, the solubility enhancer is a diol, such as pentanediol, butanediol, propane-1,3-diol, propane-1,2-diol (propylene glycol) and mixtures thereof. In particular, embodiments, the solubility enhancer is a diol, such as pentanediol, butanediol, propane-1,3-diol and propane-1,2-diol (propylene glycol). Preferably, the diol component is propylene glycol.

Other solubility enhancers that may be mentioned include polyethylene glycol, ethanol, isopropyl alcohol, glycerol and combinations thereof, some of which solubility enhancers (including ethanol and glycerol) may also act as penetration enhancers.

Preferably, the solubility enhancer component is propylene glycol.

The solubility enhancer component may be present in the compositions in an amount of from about 10% (w/w) to about 50% (w/w). More particularly, the solubility enhancer component is present in an amount of from about 10% (w/w) to about 40% (w/w), such as from about 20% (w/w) to about 40% (w/w) (e.g. about 25% (w/w) to about 35% (w/w)). In particular embodiments, the solubility enhancer component (e.g. propylene glycol) is present in an amount of 30% (w/w).

In particular embodiments, the ratio of the amounts of the penetration enhancer component (e.g. 2-(2-ethoxyethoxy)ethanol) to the solubility enhancer component (e.g. propylene glycol) penetration enhancer component:solubility enhancer component) is from about 3:1 to about 1:3, More particularly, the ratio from about 2:1 to about 1:2 (for example about 1:1).

In particular embodiments that may be mentioned, the penetration enhancer component (e.g. 2-(2-ethoxyethoxy)ethanol) and the solubility enhancer component (e.g. propylene glycol) are both present in an amount of 30% (w/w).

In particular embodiments, the compositions of the invention also comprise a gel forming polymer component. In particular, the gel forming polymer component is selected from a cellulose polymer (e.g. hydroxypropyl methyl cellulose), a cross-linked polyacrylic acid polymer and mixtures thereof. More particularly, the gel forming polymer component is hydroxypropyl methyl cellulose.

The gel forming polymer component (e.g. hydroxypropyl methyl cellulose) may be present in an amount of from about 0.05% (w/w) to about 5% (w/w), such as from about 0.05% (w/w) to about 3% (w/w), for example from about 1%(w/w) to about 3% (w/w) (e.g. from about 1% (w/w) to about 2% (w/w)).

The compositions of the invention may also comprise water and this is preferred. For gel-based compositions, the skilled person will understand that the presence of water is necessary for the composition to form a gel with the gel-forming polymer component (e.g. HPMC). Appropriate amounts of water and gel-forming polymer (and other components of the compositions) to form a gel of an appropriate viscosity for topical application to the relevant body surface (e.g. the skin) may be determined routinely by the skilled person using techniques known in the art.

In particular embodiments, compositions of the invention are in the form of a gel. Such embodiments comprise a gel-forming polymer component as defined herein (e.g. HPMC) and water to form the gel. In particular embodiments, the gel compositions have a viscosity of from about 1.0 Pa·s to about 3.0 Pa·s at a shear rate of 10⁻¹ and a temperature of about 34 °C (e.g. 34 °C), such as from about 1.5 Pa·s to about 2.5 Pa·s at a shear rate of 10⁻¹ and a temperature of about 34 °C (e.g. 34 °C), for example 1.8 Pa·s to about 2.2 Pa·s at a shear rate of 10⁻¹ and a temperature of about 34 °C (e.g. 34 °C).

Accordingly, in particular embodiments, water is included in the compositions in an amount sufficient to provide a gel-based composition with a viscosity of from about 1.0 Pa·s to about 3.0 Pa·s at a shear rate of 10⁻¹ and a temperature of about 34 °C (e.g. 34 °C)_, such as from about 1.5 Pa·s to about 2.5 Pa·s at a shear rate of 10⁻¹ and a temperature of about 34 °C (e.g. 34 °C), for example 1.8 Pa·s to about 2.2 Pa·s at a shear rate of 10⁻¹ and a temperature of about 34 °C (e.g. 34 °C).

The viscosity of the compositions may be determined by standard methods known to the skilled person and may particularly be determined using a shear rheometer (e.g a Kinexus Pro Rheometer). More particularly, the shear rheometer has a measuring geometry CP4/40, stainless steel cone:plate geometry, 40 mm diameter with 4° cone.

In particular embodiments, the compositions of the invention further comprise a lower alcohol as an additional solubiliser. The lower alcohol may be selected from ethanol, isopropanol, propanol and mixtures thereof. Preferably, the lower alcohol is isopropanol. In particular embodiments, the lower alcohol is present in an amount of from about 0.5% (w/w) to about 10% (w/w), more particularly from about 0.5% (w/w) to about 5% (w/w), such as from about 0.5% (w/w) to about 4% (w/w), for example from about 0.5% (w/w) to about 3.5% (w/w), e.g. from about 0.5% (w/w) to about 2.5% (w/w).

In further embodiments, the compositions contain an alcohol selected from the group consisting of benzyl alcohol and a lower alcohol (such as ethanol, isopropanol, propanol and mixtures thereof (e.g.isopropanol) as an additional solubiliser. In particular embodiments, the alcohol is present in an amount of from about 0.5% (w/w) to about 10% (w/w), more particularly from about 0.5% (w/w) to about 5% (w/w), such as from about 0.5% (w/w) to about 4% (w/w), for example from about 0.5% (w/w) to about 3.5% (w/w), e.g. from about 0.5% (w/w) to about 2.5% (w/w).

In particular embodiments, the additional alcohol is benzyl alcohol.

The compositions of the invention may also contain other polymer components as thickening agents. Examples of such polymers include PEG1500, PEG6000 and PEG35000.

The pH of the compositions may also be adjusted to an appropriate value, for example in the range of pH 3 to pH 6, which may vary depending on the salt form of N-[(15)-1-(4-tert-butylphenyl)ethyl]-2-(6,7-difluoro-1H-benzimidazol-1-yl)acetamide used in the composition. In particular embodiments, the pH of the compositions may be in the range of from about pH 2 to about pH 6, such as from about pH 3 to about pH 5, for example from about pH 3 to about pH 4 (e.g. about pH 3).

The compositions of the invention may also contain pH modifiers, which may be organic or inorganic acids or bases. Examples of pH modifiers include sodium hydroxide, hydrochloric acid and citric acid. In particular, the pH modifier may be sodium hydroxide.

It has also been found that the inclusion of an aminopolycarboxylic acid sequestering agent, such as EDTA, improves the stability of the active ingredient within the compositions of the invention. Accordingly, in particular embodiments, the compositions of the invention further comprise an aminopolycarboxylic acid sequestering agent, or a pharmaceutically acceptable salt thereof, in an amount of from about in an amount of from about 0.001%(w/w) to about 0.5% (w/w), more particularly from about 0.001% (w/w) to about 0.1% (w/w), or from about 0.005% (w/w) to about 0.5% (w/w), for example from about 0.005% (w/w) to about 0.05% (w/w), such as from about 0.005% (w/w) to about 0.02% (w/w) (e.g about 0.01% (w/w)). Particular aminopolycarboxylic acid sequestering agents that may be mentioned include ethylenediaminetetraacetic acid (EDTA), diethylenetriaminepentaacetic acid (DTPA; pentetic acid) and nitrilotriacetic acid (NTA) (and pharmaceutically-acceptable salts thereof). More particularly, the aminopolycarboxylic acid sequestering agent is EDTA, or a pharmaceutically acceptable salt thereof (such as a sodium salt (disodium or tetrasodium) or a sodium and calcium salt (disodium calcium edetate), which may particularly be present in an amount of from about 0.001%(w/w) to about 0.1% (w/w), for example, 0.005% (w/w) to about 0.05% (w/w), such as from about 0.005% (w/w) to about 0.02% (w/w) (e.g about 0.01% (w/w)).

Particular compositions of the invention that may be mentioned include a pharmaceutical composition (formulated/packaged and presented for topical use) comprising:
i) the compound N-[(1S)-1-(4-tert-butylphenyl)ethyl]-2-(6,7-difluoro-1H-benzimidazol-1-yl)acetamide, (optionally in the form of a pharmaceutically acceptable salt) in an amount of from about 0.05% (w/w) to about 10% (w/w) (for example, from about 0.5% (w/w) to about 5% (w/w), such as from about 0.5% (w/w) to about 2.5% (w/w) (e.g. from about 0.5% (w/w) to about 1.5% (w/w));
ii) a penetration enhancer (e.g. 2-(2-ethoxyethoxy)ethanol) in an amount of from about 10% (w/w) to about 40% (w/w) (for example from about 20% (w/w) to about 40% (w/w), e.g. about 30% (w/w)); and
iii) a solubility enhancer (e.g. propylene glycol) in an amount of from about 10%(w/w) to about 40% (w/w) (for example from about 20% (w/w) to about 40% (w/w), e.g. about 30% (w/w)).

Such compositions may optionally further comprise a gel forming component (e.g. hydroxypropyl methylcellulose) in an amount of about 0.05% (w/w) to about 5% (w/w) (such as from about 0.05% (w/w) to about 3% (w/w), for example from about 1%(w/w)) and/or a lower alcohol (e.g. isopropyl alcohol) as an additional solubiliser. Such lower alcohol is preferably present in an amount of from about 0.5% (w/w) to about 10% (w/w), more particularly from about 0.5% (w/w) to about 5% (w/w), such as from about 0.5% (w/w) to about 4% (w/w), for example from about 0.5% (w/w) to about 3.5% (w/w), e.g. from about 0.5% (w/w) to about 2.5% (w/w).

Such compositions may further comprise water (e.g. in an amount necessary to form a gel) and/or an aminopolycarboxylic acid sequestering agent, or pharmaceutically acceptable salt thereof, such as ethylenediamine tetraacetic acid, or a pharmaceutically acceptable salt thereof, (such as a sodium salt (disodium or tetrasodium) or a sodium and calcium salt (disodium calcium edetate), which may particularly be present in an amount of from about 0.001%(w/w) to about 0.1% (w/w), more particularly from about 0.005% (w/w) to about 0.05% (w/w), such as from about 0.005% (w/w) to about 0.02% (w/w) (e.g about 0.01% (w/w)).

Further compositions of the invention that may be mentioned, include a pharmaceutical composition formulated (and/or packaged or presented) for topical use (e.g in the form of a gel), wherein the composition comprises:
i) N-[(1S)-1-(4-tert-butylphenyl)ethyl]-2-(6,7-difluoro-1H-benzimidazol-1-yl)acetamide in an amount of from about 0.05% (w/w) to about 10% (w/w) (for example, from about 0.5% (w/w) to about 5% (w/w), such as from about 0.5% (w/w) to about 2.5% (w/w) (e.g. from about 0.5% (w/w) to about 1.5% (w/w)), or a pharmaceutically acceptable salt thereof;
ii) a penetration enhancer component, selected from the list consisting of 2-(2-ethoxyethoxy)ethanol, dimethyl isosorbide, glycerol, ethanol and combinations thereof (e.g. 2-(2-ethoxyethoxy)ethanol), in an amount of from about 10% (w/w) to about 50% (w/w) (for example from about 20% (w/w) to about 40% (w/w), e.g. about 30% (w/w)); and
iii) a solubility enhancer component, selected from the group consisting of pentanediol, butanediol, propane-1,3-diol, propylene glycol and mixtures thereof, (e.g. propylene glycol), in an amount of from about 10% (w/w) to about 50% (w/w) (for example from about 20% (w/w) to about 40% (w/w), e.g. about 30% (w/w)),
wherein the ratio of the penetration enhancer component: solubility enhancer component is from about 3: 1 to about 1:3.

In particular embodiments, the penetration enhancer component is selected from the group consisting of 2-(2-ethoxyethoxy)ethanol, dimethyl isosorbide and mixtures thereof; optionally the penetration enhancer component is 2-(2-ethoxyethoxy)ethanol.

In further particular embodiments, the solubility enhancer component is propylene glycol.

In further particular embodiments, the penetration enhancer component and solubility enhancer component are each present in an amount of from about 25% (w/w) to about 35% (w/w).

In further particular embodiments, the composition further comprises a gel forming polymer component, selected from the group consisting of a cellulose polymer (e.g. hydroxypropyl methyl cellulose), a cross-linked polyacrylic acid polymer and mixtures thereof, in an amount of from about 1% (w/w) to about 3% (w/w); and/or the composition further comprises a lower alcohol selected from ethanol, isopropanol, propanol, and mixtures thereof (e.g. isopropanol), which lower alcohol is optionally present in an from about 0.5% (w/w) to about 10% (w/w), more particularly from about 0.5% (w/w) to about 5% (w/w), such as from about 0.5% (w/w) to about 4% (w/w), for example from about 0.5% (w/w) to about 3.5% (w/w), e.g. from about 0.5% (w/w) to about 2.5% (w/w).

Such compositions may further comprise an aminopolycarboxylic acid sequestering agent, or a pharmaceutically acceptable salt thereof, in an amount of from about 0.001%(w/w) to about 0.5% (w/w), more particularly from about 0.001% (w/w) to about 0.1% (w/w), or from about 0.005% (w/w) to about 0.5% (w/w), for example from about 0.005% (w/w) to about 0.05% (w/w), such as from about 0.005% (w/w) to about 0.02% (w/w) (e.g about 0.01% (w/w)). Particular aminopolycarboxylic acid sequestering agents that may be mentioned include ethylenediaminetetraacetic acid (EDTA), diethylenetriaminepentaacetic acid (DTPA; pentetic acid) and nitrilotriacetic acid (NTA) (and pharmaceutically-acceptable salts thereof). More particularly, the aminopolycarboxylic acid sequestering agent is EDTA, or a pharmaceutically acceptable salt thereof (such as a sodium salt (disodium or tetrasodium) or a sodium and calcium salt (disodium calcium edetate), which may particularly be present in an amount of from about 0.001%(w/w) to about 0.1% (w/w), more particularly from about 0.005% (w/w) to about 0.05% (w/w), such as from about 0.005% (w/w) to about 0.02% (w/w) (e.g about 0.01% (w/w)).

Such compositions may particularly be in the form of a gel (and therefore also contain water).

Further particular compositions of the invention that may be mentioned, include a composition formulated (and/or packaged or presented for) topical use (e.g. in the form of a gel) comprising:
i) N-[(1S)-1-(4-tert-butylphenyl)ethyl]-2-(6,7-difluoro-1H-benzimidazol-1-yl)acetamide in an amount of from about 0.5% (w/w) to about 3% (w/w) ) (such as from about 0.5% (w/w) to about 2.5% (w/w) (e.g. from about 0.5% (w/w) to about 1.5% (w/w)), or a pharmaceutically acceptable salt thereof;
ii) 2-(2-ethoxyethoxy)ethanol in an amount of from about 25% (w/w) to about 35% (w/w);
iii) propylene glycol in an amount of from about 25% (w/w) to about 35% (w/w);
iv) isopropyl alcohol in an amount of from about 0.5% (w/w) to about 3.5% (w/w);
v) hydroxypropyl methyl cellulose in an amount of from about 1% (w/w) to about 3% (w/w)
vi) water (e.g. in an amount sufficient to form a gel with the hydroxypropyl methyl cellulose).

Such compositions may further comprise an aminopolycarboxylic acid sequestering agent, or pharmaceutically acceptable salt thereof, such as ethylenediamine tetraacetic acid, or a pharmaceutically acceptable salt thereof, (such as a sodium salt (disodium or tetrasodium) or a sodium and calcium salt (disodium calcium edetate), which may particularly be present in an amount of from about from about 0.001%(w/w) to about 0.1% (w/w), more particularly from about 0.005% (w/w) to about 0.05% (w/w), such as from about 0.005% (w/w) to about 0.02% (w/w) (e.g about 0.01% (w/w)).

Further compositions of the invention that may be mentioned, include a composition formulated (and/or packaged or presented for) topical use (e.g. in the form of a gel), comprising:
i) N-[(1S)-1-(4-tert-butylphenyl)ethyl]-2-(6,7-difluoro-1H-benzimidazol-1-yl)acetamide, or a pharmaceutically acceptable salt thereof, in an amount of from about 0.5% (w/w) to about 3% (w/w)), (such as from about 0.5% (w/w) to about 2.5% (w/w) (e.g. from about 0.5% (w/w) to about 1.5% (w/w));
ii) 2-(2-ethoxyethoxy)ethanol in an amount of from about 25% (w/w) to about 35% (w/w);
iii) propylene glycol in an amount of from about 25% (w/w) to about 35% (w/w);
iv) hydroxypropyl methyl cellulose in an amount of from about 1% (w/w) to about 3% (w/w)
v) water (e.g. in an amount sufficient to form a gel with the hyroxypropyl methyl cellulose).

Such compositions may further comprise an aminopolycarboxylic acid sequestering agent, or pharmaceutically acceptable salt thereof, such as ethylenediamine tetraacetic acid, or a pharmaceutically acceptable salt thereof, (such as a sodium salt (disodium or tetrasodium) or a sodium and calcium salt (disodium calcium edetate), which may particularly be present in an amount of from about 0.001%(w/w) to about 0.1% (w/w), more particularly from about 0.005% (w/w) to about 0.05% (w/w), such as from about 0.005% (w/w) to about 0.02% (w/w) (e.g about 0.01% (w/w)).

### Medical uses

In accordance with the first aspect of the invention, the compositions of the invention are used in the treatment of pain.

The skilled person will understand that references to the treatment of pain (or, similarly, to treating pain) will take its normal meaning in the field of medicine. In particular, the terms may refer to achieving a reduction in the severity and/or frequency of occurrence of pain, as adjudged by a physician attending a patient having or being susceptible to pain. For example, in the case of pain, the term may refer to a reduction in the severity of the pain experienced by the patient as determined/described with reference to an appropriate scale (e.g. a numeric rating scale or a visual analogue scale).

For the avoidance of doubt, the treatment of pain includes the treatment of recognised pain categories (e.g. nociceptive pain or neuropathic pain) in a general sense, including, in particular, pain amenable to topical treatment, and also to treating the pain associated with specific disorders (such as those listed herein) for which pain is a symptom.

As used herein, references to a patient (or to patients) will refer to a living subject being treated, including mammalian (e.g. human) patients. In particular, references to a patient will refer to human patients.

For the avoidance of doubt, the skilled person will understand that such treatment or prevention will be performed in a patient (or subject) in need thereof. The need of a patient (or subject) for such treatment may be assessed by those skilled the art using routine techniques.

As used herein, the terms disease and disorder (and, similarly, the terms condition, illness, medical problem, and the like) may be used interchangeably.

As used herein, the term effective amount will refer to an amount of a compound that confers a therapeutic effect on the treated patient. The effect may be observed in a manner that is objective (i.e. measurable by some test or marker) or subjective (i.e. the subject gives an indication of and/or feels an effect). In particular, the effect may be observed (e.g. measured) in a manner that is objective, using appropriate tests as known to those skilled in the art.

The compositions of the invention are used to treat pain by topical administration. Therefore, the pain to be treated could generally be defined as pain that is amenable to topical treatment. As the compositions are applied topically, the active ingredient does not enter systemic circulation.

The compositions of the invention may be applied topically to different body surfaces in the treatment of pain. In particular, the compositions may be applied to a mucosal surface, the eyes or the skin.

In preferred embodiments, the body surface to which the compositions are topically applied is the skin (and, more particularly, intact skin).

In particular embodiments, the compositions are applied to intact skin, which may be beneficial because it limits systemic exposure. Applying a topical composition to broken or injured skin can lead to systemic exposure, which may, in some circumstances, be undesirable (for example because it may lead to undesirable side effects).

In particular embodiments, the pain to be treated topically with the compositions of the invention is nociceptive pain (and, particularly, acute nociceptive pain).

In further particular embodiments, the pain is associated with a dermatological pain condition. Examples of such conditions include radiotherapy-induced pain, pyoderma, gangrenosum, hidradenitis suppurativa, calciphylaxis, vasculopathies, burn injury, shingles, scleroderma and dermatomyositis. Such conditions are believed to be amenable to treatment by topical application of a composition of the invention to the skin.

In further embodiments, the pain associated with enthesopathy. Examples of enthesopathies include tendonitis (e.g. Achilles tendonitis), tennis elbow and mouse arm.

In further embodiments, the pain is inflammatory pain, including pain associated with rheumatoid arthritis.

In further embodiments, the pain is neuropathic pain, and, particularly, peripheral, neuropathic pain, and this is preferred.

In particular embodiments, the peripheral neuropathic pain is selected from the group consisting of postherpetic neuropathy, post-traumatic neuropathy, post-operative neuropathic pain, painful diabetic polyneuropathy, HIV neuropathy, chemotherapy induced neuropathic pain, leprosy neuropathic pain, post amputation pain.

In particular embodiments, the peripheral neuropathic pain is postherpetic neuropathy.

In particular embodiments, the peripheral neuropathic pain is post-traumatic neuropathy.

In particular embodiments, the peripheral neuropathic pain is post-operative neuropathic pain.

In particular embodiments, the peripheral neuropathic pain is painful diabetic polyneuropathy.

In particular embodiments, the peripheral neuropathic pain is HIV neuropathy.

In particular embodiments, the peripheral neuropathic pain is chemotherapy induced neuropathic pain.

In particular embodiments, the peripheral neuropathic pain is leprosy neuropathic pain.

In particular embodiments, the peripheral neuropathic pain is post amputation pain.

### Dosage forms and dosages

In particular embodiments, the compositions of the invention are in a dosage form selected from the list consisting of a cream, a (topical) (e.g. liquid) spray, a (topical) patch, a gel, an ointment, a lotion and a paste. More particularly, the dosage form is a cream, (topical) (e.g. liquid) spray, (topical) patch or gel.

In particular embodiments, the pharmaceutical composition is in the form of a cream.

In particular embodiments, the pharmaceutical composition is in the form of a (topical) (e.g. liquid) spray.

In particular embodiments, the pharmaceutical composition is in the form of a (topical) patch.

In particular embodiments, the pharmaceutical composition is in the form of a gel.

In addition to the specific excipients discussed above, the different dosage forms may contain particular excipients appropriate to each form, as follows.
- suitable excipients for gel formulations include matrix materials including cellulose derivatives, carbomer and alginates, gummi tragacanthae, gelatin, pectin, carrageenan, gellan gum, starch, Xanthan gum, cationic guar gum, agar, noncellulosic polysaccharides, saccharides such as glucose, glycerin, propanediol, vinyl polymers, acrylic resins, polyvinyl alcohol, carboxyvinyl polymer and, particularly, hyaluronic acid);
- suitable excipients for pastes and ointments include glycerin, vaseline, paraffin, polyethylene glycols of different molecular weights, etc.);
- suitable excipients for creams include hydroxypropyl methyl cellulose, gelatin, polyethylene glycols of different molecular weights, sodium dodecyl sulfate, sodium fatty alcohol polyoxyethylene ether sulfonate, corn gluten powder and acrylamide);
- suitable excipients for liquid, for example, water (aerosol) sprays include viscosity modifiers, such as hyaluronic acid, sugars, such as glucose and lactose, emulsifiers, buffering agents, alcohols, water, preservatives, sweeteners, flavours, etc.);

Further pharmaceutically acceptable excipients include moisturizing agents, such as glycerol, glycerin, polyethylene glycol, trehalose, glycerol, petrolatum, paraffin oil, silicone oil, hyaluronic acid and salts (e.g. sodium and potassium salts) thereof, octanoic/caprylic triglyceride, and the like; and/or antioxidants, such as vitamins and glutathione; and/or pH modifiers, such as acids, bases and pH buffers, may also be included in such formulations, as appropriate. Furthermore, surfactants/emulsifiers, such as hexadecanol (cetyl alcohol), fatty acids (e.g. stearic acid), sodium dodecyl sulfate (sodium lauryl sulfate), sorbitan esters (e.g. sorbitan stearate, sorbitan oleate, etc.), monoacyl glycerides (such as glyceryl monostearate), polyethoxylated alcohols, polyvinyl alcohols, polyol esters, polyoxyethylene alkyl ethers (e.g. polyoxyethylene sorbitan monooleate), polyoxyethylene castor oil derivatives, ethoxylated fatty acid esters, polyoxylglycerides, lauryl dimethyl amine oxide, bile salts (e.g. sodium deoxycholate, sodium cholate), lipids (e.g. fatty acids, glycerolipids, glycerophospholipids, sphingolipids, sterols, prenols, saccharolipids, polyketides), phospholipids, N,N-dimethyldodecylamine-N-oxide, hexadecyltrimethyl-ammonium bromide, poloxamers, lecithin, sterols (e.g. cholesterol), sugar esters, polysorbates, and the like; preservatives, such as phenoxyethanol, ethylhexyl glycerin, and the like; and thickeners, such as acryloyldimethyltaurate/VP copolymer, may be included. In particular, stearic acid, glyceryl monostearate, hexadecanol, sorbitan stearate, cetyl alcohol, octanoic/capric glyceride etc. may be included, particularly in cream formulations.

Compositions of the invention may further be combined with an appropriate matrix material to prepare a dressing or a therapeutic patch for application on a biological surface, such as the skin or a mucosal surface (particularly the skin). Such formulations may thus be employed to impregnate a matrix material, such as gauze, non-woven cloth or silk paper. The therapeutic patch may alternatively be, for example, a band-aid, a facial mask, an eye mask, a hand mask, a foot mask, etc.

In particular embodiments, the compositions of the invention contain the compound N-[(1S)-1-(4-tert-butylphenyl)ethyl]-2-(6,7-difluoro-1H-benzimidazol-1-yl)acetamide (calculated as the free (non salt compound) in an amount of from about 5 mg/mL to about 100 mg/mL. More particularly, the compound may be present in an amount of 5 mg/mL to about 70 mg/mL, such as from about 5 mg/mL to about 50 mg/mL, for example from about 5 mg/mL to about 30 mg/mL, such as from about 5 mg/mL to about 30 mg/mL or from about 5 mg/mL to about 25 mg/mL (e.g. from about 5 mg/mL to about 15 mg/mL). In further embodiments, the compound N-[(1S)-1-(4-tert-butylphenyl)ethyl]-2-(6,7-difluoro-1H-benzimidazol-1-yl)acetamide is present in an amount of from about 5 mg/mL to about 20 mg/mL, such as from about 10 mg/mL to about 20 mg/mL.

In accordance with the invention, the compositions may be applied to the body surface (e.g. skin) in an amount of from about 0.01 mL/cm² to about 2.0 mL/cm². More particularly, the composition is applied in an amount of from about 0.02 mL/cm² to about 1 mL/cm², such as from about 0.02 mL/cm² to about 0.2 mL/cm2 (e.g. from about 0.02 mL/cm² to about 0.1 mL/cm²). Yet more particularly, the composition is applied to the body surface in an amount of from about 0.01 mL/cm² to about 0.06 mL/cm², such as from about 0.01 mL/cm² to about 0.05 mL/cm² (for example from about 0.01 to about 0.03 mL/cm²). In particular embodiments, the composition is applied in an amount of 0.05 mL/cm².

In further embodiments, the treatment comprises applying the composition in an amount to give a dose of the compound N-[(1S)-1-(4-tert-butylphenyl)ethyl]-2-(6,7-difluoro-1H-benzimidazol-1-yl)acetamide (calculated as the free (non-salt) compound) of from about 50 µg/cm² to about 1000 µg/cm². More particularly, the dose of the compound is from about 100 µg/cm² to about 900 µg/cm² about 200 µg/cm² to about 900 µg/cm², such as from about 300 µg/cm² to about 900 µg/cm² (e.g. from about 500 µg/cm² to about 900 µg/cm²). In particular embodiments, the composition is administered in an amount sufficient to give a dose of about 700 µg/cm². In further embodiments, the composition is administered in an amount sufficient to give a dose of the active compound of from about 100 µg/cm² to about 700 µg/cm².

In further embodiments, the compositions are applied in an amount sufficient to give a dose of the compound (in free (non-salt) form) of from about 50 µg/cm² to about 600 µg/cm², such as from about 100 µg/cm² to about 600 µg/cm², for example from about 100 µg/cm² to about 500 µg/cm² (e.g. from about 100 µg/cm² to about 400 µg/cm² or from about 100 µg/cm² to about 300 µg/cm² or from about 50 µg/cm² to about 400 µg/cm² or from about 50 µg/cm² to about 300 µg/cm²).

In particular embodiments, the total amount of the composition applied to the body surface is from about 50 mg to about 1 g (per application), such as from about 50 mg to about 700, for example, from about 50 mg g to about 500 mg (e.g. from about 100 mg to about 500 mg or about 200 mg to about 500 mg).

The compositions of the invention have been shown to have a long-lasting analgesic effect. Therefore, treatment with the compositions of the invention may require the composition to be administered less frequently that other pain medications. Accordingly, in further embodiments of the invention, the treatment comprises administering the composition once or, preferably, twice a day.

The compositions may also be administered between one and four times a day.

### Combinations and kits-of-parts

The skilled person will understand that treatment with compositions of the invention may further comprise (i.e. be combined with) further treatment(s) for the treatment of pain. In particular, treatment with compounds of the invention may be combined with means for the treatment of pain (such as peripheral neuropathic pain as described herein), such as treatment with one or more other therapeutic agent that is useful in the in the treatment of pain.

As described herein, the compositions of the invention may be combined with one or more other (i.e. different) therapeutic agents that are useful in the treatment of pain. Such combination products provide for the topical administration of a composition of the invention in conjunction with treatment with one or more other therapeutic agent, and may be presented either as separate compositions, wherein at least one of the compositions is a composition of the invention, and at least one comprises another therapeutic agent, or may be presented (i.e. formulated) as a combined preparation.

Accordingly, in a further embodiment, the compositions of the invention, including those for use in accordance with the invention, further comprise a second therapeutic agent for the treatment of pain.

In a further aspect of the invention, there is provided a combination product comprising:
(I) a composition of the invention and
(II) one or more other therapeutic agent that is useful in the treatment of pain.
wherein each of components (I) and (II) is formulated in admixture, optionally with one or more a pharmaceutically-acceptable excipient.

In a further aspect of the invention, there is provided a kit-of-parts comprising:
(a) a composition of the invention; and
(b) one or more other therapeutic agent that is useful in the treatment of pain (such as peripheral neuropathic pain as described herein), optionally in admixture with one or more pharmaceutically-acceptable excipient,
which components (a) and (b) are each provided in a form that is suitable for administration in conjunction (i.e. concomitantly or sequentially) with the other.

With respect to the kits-of-parts as described herein, by "administration in conjunction with" (and similarly "administered in conjunction with") we include that respective formulations are administered, sequentially, separately or simultaneously, as part of a medical intervention directed towards treatment of the relevant condition.

Thus, in relation to the present invention, the term "administration in conjunction with" (and similarly "administered in conjunction with") includes that the two active ingredients (i.e. a compound of the invention and a further agent for the treatment of pain, or compositions comprising the same) are administered (optionally repeatedly) either together, or sufficiently closely in time, to enable a beneficial effect for the patient, that is greater, over the course of the treatment of pain, than if either agent is administered (optionally repeatedly) alone, in the absence of the other component, over the same course of treatment. Determination of whether a combination provides a greater beneficial effect in respect of, and over the course of, treatment of a particular condition will depend upon the condition to be treated, but may be achieved routinely by the skilled person.

Further, in the context of the present invention, the term "in conjunction with" includes that one or other of the two formulations may be administered (optionally repeatedly) prior to, after, and/or at the same time as, administration of the other component. When used in this context, the terms "administered simultaneously" and "administered at the same time as" includes instances where the individual doses of the compound of the invention and the additional compound for the treatment of pain, or pharmaceutically acceptable salts thereof, are administered within 48 hours (e.g. within 24 hours, 12 hours, 6 hours, 3 hours, 2 hours, 1 hour, 45 minutes, 30 minutes, 20 minutes or 10 minutes) of each other.

As used herein, references to other therapeutic agents that are "useful" in the treatment of pain will refer to agents that are known to be suitable for use in that manner (e.g. agents commonly used for that purpose). Such references may therefore be replaced with references to agents "suitable for" the relevant purpose.

In a further embodiment of the compositions for use, methods and uses described herein, the treatment of pain further comprises treatment with a composition of the invention in combination with another agent suitable for the treatment of pain, such as, in particular, an orally administered pain medication.

Examples of further therapeutic agents suitable for use in combination with treatment with the compositions of the invention include opioids, non-steroidal antiinflammatories, antidepressants, anticonvulsants, NMDA antagonists and Cannabinoids.

### Preparation of compositions

In a further aspect of the invention, there is provided a process for the manufacture of a pharmaceutical composition, as hereinbefore defined, which process comprises bringing into association the compound N-[(1S)-1-(4-tert-butylphenyl)ethyl]-2-(6,7-difluoro-1H-benzimidazol-1-yl)acetamide, or pharmaceutically acceptable salt thereof, with one or more pharmaceutically-acceptable excipient.

In a particular embodiment, there is provided a process for the preparation of a gel-based formulation as described herein, comprising the steps of
i) hydration of a gel forming polymer (e.g. hydroxypropyl methylcellulose) in water;
ii) dissolving N-[(1S)-1-(4-tert-butylphenyl)ethyl]-2-(6,7-difluoro-1H-benzimidazol-1-yl)acetamide, or pharmaceutically acceptable salt thereof, in an organic phase comprising a penetration enhancer component (e.g. 2-(2-ethoxyethoxy)ethanol) and a solubility enhancer component (e.g. propylene glycol);
iii) mixing the water-based gel component with the organic phase; and
iv) optionally, adjusting the pH to an appropriate value (e.g. about pH 3), for example by the addition of a suitable base (e.g. sodium hydroxide solution).

Without wishing to be bound by theory, it is believed that the compositions, compositions for use, methods and uses described herein provide improved topical treatments for pain. In particular, topical treatment with the compositions described herein has been shown to be highly efficacious in the treatment of pain, when compared to the previous oral use of the same active ingredient and also when compared to oral treatment with a range of established pain medications. The topical administration of the compositions has also been found to be associated with a significantly longer lasting analgesic effect than oral administration of the same active ingredient.

Treatment in accordance with the methods described herein may have the advantage that it is may be more efficacious than, produce fewer side effects (including increased body core temperature and/or disturbed thermosensation) than, and/or have a better pharmacokinetic profile (e.g. higher oral bioavailability and/or lower clearance) than, and/or have other useful properties over similar treatments known in the prior art.

### Brief Description of Figures

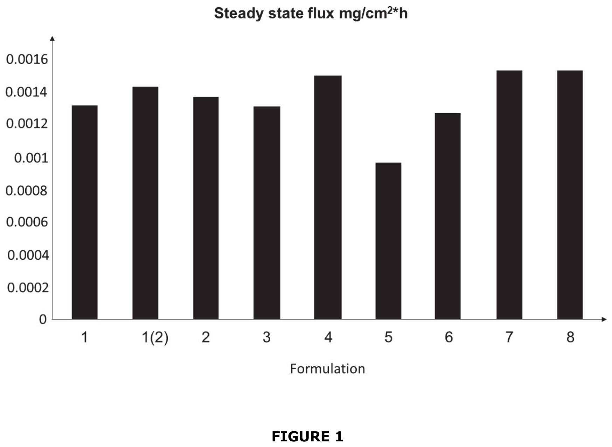

Figure 1 shows the flux of the active ingredient across the Franz Cell membrane for a range of compositions comprising N-[(1S)-1-(4-tert-butylphenyl)ethyl]-2-(6,7-difluoro-1H-benzimidazol-1-yl)acetamide and other excipients, including propylene glycol and Transcutol^{®} (2-(2-ethoxyethoxy)ethanol). Two values are shown for Formulation 1. The figure shows that high levels of flux were achieved for all formulations tested.

### Example 1 - Franz Cell Flux Experiments

The Franz Cell is a model for transdermal absorption of active ingredients from topical formulations. An appropriate acceptor solution is added to the Franz Cell apparatus, which is then fitted with a permeable membrane. The test formulation is applied to the top of the membrane the rate of diffusion through the membrane is measured overtime.

### 1.1 Comparative flux -gel formulations and solutions

Permeability measurements were taken using Permeagear vertical jacketed diffusion cells, fitted with a Silicone membrane (Silatos Silicone Sheeting Ref 7458), with an area of 0.64 cm². 5 mL 5% hydroxypropyl-beta-cyclodextrin (HPCD) in 0.1 M HCl was used as the acceptor solution, which was kept at a temperature of 32 °C. Approximately 0.5 to 0.7 g of the formulations was added to each well and 0.2 mL samples were taken at 0.25, 0.5, 1, 2, 3, 4, 14.5 and 24 hour time points.

The comparative flux through a Franz cell membrane was assessed for gel formulations N-[(1S)-1-(4-tert-butylphenyl)ethyl]-2-(6,7-difluoro-1H-benzimidazol-1-yl)acetamide (Compound 1) compared to solutions of the compound.

Details of the gel formulations are given below. The flux of the active ingredient through the membrane of the Franz Cell was compared to that achieved by 5 mg/mL solutions of Compound 1 (free base (non-salt)) solutions in Transcutol^{®} and dimethylisosorbide.
1.

| | |
|---|---|
| Compound 1 (free base) | 30 mg (15 mg/mL) |
| Transcutol^{®} (2-(2-ethoxyethoxy)ethanol) | 1 mL |
| Carbopol^{®} gel (3%) | 0.98 g |
| Propylene glycol | 0.1 mL |
| Water | qs |

2.

| | |
|---|---|
| Compound 1 (free base) | 32 mg (15 mg/mL) |
| Dimethylisosorbide | 1 mL |
| Carbopol^{®} gel (3%) | 0.98 g |
| Propylene glycol | 0.1 mL |
| Water | qs |

The results are shown in the table below.

| **Formulation** | **Flux** | |
|---|---|---|
| | **µg/h*cm²** | **mole/h*cm²** |
| Formulation 1 | 0.82 | 2.2 |
| 5 mg/mL solution in Transcutol^{®} | 0.03 | 0.1 |
| Formulation 2 | 0.64 | 1.7 |
| 5 mg/mL solution in dimethylisosorbide | 0.03 | 0.1 |

The gel formulations were found to give a significantly higher permeability than the pure solutions. The level of improvement indicates that it cannot be attributed to the increased concentration of the active ingredient alone. The presence of propylene glycol in the formulations could also contribute to increased flux.

### 1.2 Further permeability experiments

The flux of the active ingredient (Compound 1) from a range of further gel formulations comprising Transcutol^{®} and propylene glycol was assessed using Permeagear vertical jacketed diffusion cells, fitted with a PDMS Membrane SSP-M823-005, with an area of 0.64 cm². 5 mL 5% hydroxypropyl-beta-cyclodextrin (HPCD) in 0.1 M HCl was used as the acceptor solution, which was kept at a temperature of 32 °C. Approximately 0.5 to 0.7 g of the formulations was added to each well and 0.2 mL samples were taken at 0.25, 0.5, 1, 2, 3, 4, 18.5/20 and 24 hour time points.

The formulations tested are described in the table below. Formulation 2 contained the active ingredient as the parent compound and all other formulations contained the active ingredient in the form of a hydrogen sulfate salt (the amounts quoted refer to the amount of the active ingredient (parent compound) present)

| **Formulation** | **Active ingredient** | **Other components (w/w)** |
|---|---|---|
| 1 | 14 mg/g | 30% propylene glycol, 30% Transcutol^{®}, 2% benzyl alcohol, 1.4% HPMC, NaOH (q.s. pH 3), water (to 100%) |
| 2¹ | 14 mg/g | 30% propylene glycol, 30% Transcutol^{®}, 2% benzyl alcohol, 1.4% HPMC⁴, NaOH (q.s. pH 6), water (to 100%) |
| 3² | 20 mg/g | 30% propylene glycol, 30% Transcutol^{®}, 2% benzyl alcohol, 1.4% HPMC, NaOH (q.s. pH 3) water (to 100%) |
| 4 | 14 mg/g | 30% propylene glycol, 30% Transcutol^{®}, 0.8% isopropyl alcohol, 1.4% HPMC, NaOH (q.s. pH 3), water (to 100%) |
| 5 | 7 mg/g | 30% propylene glycol, 30% Transcutol^{®}, 1.4% HPMC, NaOH (q.s. pH 3), water (to 100%) |
| 6 | 14 mg/g | 32% propylene glycol, 30% Transcutol^{®}, 2% isopropyl alcohol, 0.6% HPMC, NaOH (q.s pH 3) water (to 100%) |
| 7 | 14 mg/g | 30% propylene glycol, 30% Transcutol^{®}, 2% isopropyl alcohol, NaOH (q.s. pH 3), water (to 100%) |
| 8³ | 14 mg/g | 30% propylene glycol, 30% Transcutol^{®}, 2% isopropyl alcohol, 1.2% HPMC, NaOH (q.s. pH 3), water (to 100%) |

| | | |
|---|---|---|
| ¹Parent compound used; active ingredient not fully dissolved ²Active ingredient not fully dissolved ³Formulation selected for clinical study ⁴HPMC = hydroxypropyl methylcellulose | | |

The flux of the active ingredient across the membrane was very similar for all formulations tested. The results are summarized in Figure 1.

### Example 1.3 Preparation of the clinical trial formulation

The following composition was selected for use in the clinical study described in Example 2.

| **Component** | **Amount** |
|---|---|
| N-[(1S)-1-(4-tert-butylphenyl)ethyl]-2-(6,7-difluoro-1H-benzimidazol-1-yl)acetamide.H₂SO₄ | 17.7 mg/g (14 mg/g active agent) |
| 2-(2-ethoxyethoxy)ethanol | 300 mg/g |
| Propylene glycol | 300 mg/g |
| Isopropyl alcohol | 20 mg/g |
| Hydroxypropyl methylcellulose (HPMC) | 12 mg/g |
| Sodium Hydroxide | q.s to pH 3 |
| Water | To 1g |

The composition was prepared according to the following process:
i) The HPMC polymer was hydrated in water (4.0% w/w HPMC polymer in water) by dispersing the polymer powder in water at 80 °C with vigorous stirring and cooling the dispersion to form a clear viscous gel.
ii) The active ingredient was dissolved in a mixture of the organic excipients propylene glycol, Transcutol^{®} (2-(2-ethoxyethoxy)ethanol) and isopropyl alcohol to give the organic phase.
iii) Mixing the pre-hydrated polymer gel and the organic phase by slowly adding the organic phase to the polymer gel with vigorous stirring and shaking.
iv) Adjusting the pH of the composition with the addition of sodium hydroxide solution.

The viscosity of the composition was determined at shear rates of 10⁻¹ and 100⁻¹ at a temperature of 34 °C using a Kinexus Pro Rheometer with measuring geometry CP4/40, stainless steel cone:plate geometry, 40 mm diameter with 4° cone. The viscosity was 2.1 Pa·s at a shear rate of 10⁻¹ and 0.51 Pa·s at a shear rate of 100⁻¹.

### Example 2 - Clinical study

The efficacy of a gel formulation of N-[(1S)-1-(4-tert-butylphenyl)ethyl]-2-(6,7-difluoro-1H-benzimidazol-1-yl)acetamide (as a hydrogen sulfate salt) (hereinafter, 'the Compound 1 Gel') were assessed in a double-blind, randomized placebo-controlled study on normal skin, skin optimized for penetration and skin exposed to ultraviolet B radiation in 24 healthy volunteers. Details of the formulation used are given in the table below.

| **Component** | **Amount** |
|---|---|
| N-[(1S)-1-(4-tert-butylphenyl)ethyl]-2-(6,7-difluoro-1H-benzimidazol-1-yl)acetamide.H₂SO₄ | 17.7 mg/g (14 mg/g active agent) |
| 2-(2-ethoxyethoxy)ethanol | 300 mg/g |
| Propylene glycol | 300 mg/g |
| Isopropyl alcohol | 20 mg/g |
| Hydroxypropyl methylcellulose (HPMC) | 12 mg/g |
| Sodium Hydroxide | q.s to pH 3 |
| Water | To 1g |

Healthy volunteers were screened for eligibility from Day -28 to Day -2 before first treatment. During screening also, a training session (without study medication) was performed in order to introduce subjects to the testing and rating procedures. Prior to testing the laser evoked potentials (LEPs) from healthy skin, the individual Laser Pain Threshold (LPT) was be determined for each volunteer.

At Day 1, subjects that signed the informed consent and were eligible for the study, were irradiated with increasing dosages of UVR to evaluate their individual minimal erythema dose (MED) on naevi free test areas. At Day 2, read out to determine MED will be done.

Six treatment areas with a size of 20 cm² were defined on Day 1. Area 1 and 2 were defined for treatment on normal skin. Area 3 and 4 were defined to examine maximized application conditions using skin stripping to remove parts of the stratum corneum using a standardized stripping protocol with adhesive tape and occlusive application. Area 5 and 6 were reserved for exposure with UVR. The areas 1-6 were randomized to treatment with either the Compound 1 Gel or placebo (three areas each). The placebo formulation was the same as Compound 1 Gel with the active ingredient removed and citric and hydrochloric acid added as pH modifiers.

Treatment was performed once daily for 5 consecutive days, 3 times in the pre-UVR part of the study, once before UVR at Day 4 and once 24 h after UVR at Day 5.

At Day 1, Areas 1 -4 were treated with 1ml of the Compound 1 Gel (0.05 ml/cm²) or placebo and were tested for changes in LEP-amplitudes and Visual Analog Scale Rating of Pain (VAS-P). Assessments were performed before and after (investigational medicinal product) IMP application. At Days 2 and 3, LEPs and VAS-P assessments will also be performed before and 1 h after the second and third application of IMP at the same time as on Day 1 (±1h). At test Day 4, LEPs and VAS-P were evaluated for areas 1-4 at 1, 6 and 9 h after the 4th IMP application.

At the 2 h time point on Day 4, skin areas 5 and 6 will be UVR irradiated with 2 MED. LEPs, VAS-P, erythema (by skin reflection spectrometry (SRS)) and pin prick hyperalgesia (by weighted needle threshold (WNT)) were evaluated before and 1, 2, 3, 6 and 9 h after UVR. At the same time points, anti-inflammatory effects on the areas of UVR will be evaluated by SRS with the a-value being the measure of "redness". Subjects returned on Day 5 for the 5th IMP application. One hour after IMP application and 24±1h after UVR, LEPs and VAS-Pain were evaluated on areas 1-6. In addition, erythema (SRS) and pin prick hyperalgesia (WNT) were evaluated on areas 5 and 6. Subjects returned for a follow-up visit at Day 9 (±1).

### Methods for evaluating efficacy

Efficacy was assessed by the following methods:

### (i) Laser algesimetry

Laser algesimetry is an experimental induction of pain by means of a contact-free thermo-nociceptive laser beam with a constant short duration (ms) and individually adjusted intensity above the pain threshold (determined at Screening). Objective and quantitative measurement of nociception is accomplished by the analysis of the contingent vertex EEG event-related potentials (somatosensory/radiant heat ERPs). Analgesic and anti-hyperalgesic properties of drugs can be demonstrated objectively and quantitatively by alterations of the LEP parameters, primarily by reductions of the amplitudes of main LEP components (N2 and P2) versus placebo - using Peak-to-Peak amplitude (PtP).

The study effects of the study medications (the Compound 1 Gel and placebo) were measured as a reduction in the PtP amplitude.

### (ii) VAS-Pain

VAS-Pain is a subjective assessment of pain, which was used for all skin conditions. Electronic nociceptive scoring with electronic 100-mm visual analog scales (VASs) for post-laser pain (VAS-P) was assessed on a tablet PC, which allowed the subject to discriminate pain severity between no pain and strong pain using a 0 to 100 mm scale. The measurements were taken after each laser session - summarizing the overall impression of the "painfulness" of the whole session.

### (iii) WNT Measurement (mechanical hyperalgesia)

The WNT investigation of mechanical hyperalgesia was performed on the UVB irradiated treatment areas using fixed weight steps at the same session time points as done with LEPs+VAS-P (always after these sessions). Skin contact was made by a rounded/blunt needle tip placed (with defined weight ranging from 1 to 512 mN) on the skin. The WNT set was supplied by Institute of Physiology and Pathophysiology of University Erlangen-Nuremberg in a calibrated status [Rolke et al 2006]). Subjects had to indicate with which weight they feel pain (threshold) vs. pressure in the respective treatment area.

### (iv) Skin Reflection Spectrometry

Quantitative measurement of UV-erythema intensity was done by a CE-labeled Chroma Meter CR-400 (Konica-Minolta Optics, Inc. Munich) - looking for spectral shifts, respectively for changes of the colorimetric parameters - according to the CIE-Lab system (CIE = Commission Internationale l'Éclairage, Lab = Color dimension for SRS, L = luminescence, a = red/green dimension, b = blue/yellow dimension). 'Cold' polychromatic light was guided to the skin to a photomultiplier. Output variables are colorimetric parameters according to this CIE Lab system - in this case the a-value ("redness") - a parameter without any dimension. An anti-inflammatory effect of drugs results in a lower a-value of Lab measurement system (= less redness).

### Method of Ultraviolet-B Exposure of the Skin

The UVB model is used as a standard for the investigation of anti-inflammatory and analgesic compounds.

The UV light is composed predominantly of UVB radiation (280 320 nm of invisible spectral range, with a narrow-band emission at 311 nm), which is known to distinctly enhance redness (erythema) of skin, more than UVA radiation that has predominantly skin tanning properties.

At Screening, UVB was applied in different doses once to 6 small areas (1 × 1 cm2 each = 6 cm2) of the skin on the subjects' backs, in order to determine the individual minimum dose which produces a clearly discernible erythema - the minimal erythema dose (MED). The UV radiation was provided using a Dermalight^{®} 80 narrow-band UVB source (311 nm, invisible range), which contains Philips TL 9W/01 UVB tubes to produce the UV light. The first area with a regular and well-defined square erythema (after a development time of 6 to 8 hours at least) will defined as the individual MED.

In the morning of UVR Day, the 2-fold individual MED was applied using the Dermalight 180 narrow-band UVB source (from 310-315 nm, with mean spectrum at 311 nm, invisible range) manufactured by A.L.T. Lichttherapietechnik GmbH, Zörbig, Germany and marketed by Dr. K. Hönle Medizintechnik GmbH, Kaufering, Germany. The UVB exposure will be performed on two skin areas of the back (2 areas of 5 × 4 cm = 20 cm2 each) to produce a homogenous area of skin erythema and hyperalgesia that is large enough to perform repeated laser measurements.

### Sequence of efficacy tests

Treatment areas were tested in numerical sequence (1,2,3,4,5,6).

For an individual treatment area, efficacy parameters were evaluated in the following sequence:
1. SRS (UVR areas only)
2. WNT (UVR area only)
3. LEP
4. VAS Pain

### Efficacy variables

Laser Evoked Potential
   - N2-P2 Peak-to-Peak (PtP) Amplitude (µV)
Pain
   - VAS Pain (mm)
Mechanical hyperalgesia
   - Weighted needle testing (mN)
Erythema
   - Skin Reflection Spectrometry

### Endpoint analysis

The statistical analysis was based on the evaluation of treatment related differences at the individual time points and comparison of the AUC (Area Under the Curve) parameter derived from the efficacy endpoints.

The timepoints to be included in the calculation of the AUC depended on the objective.
1. For evaluating objective a) and b) (treatment areas 1-4), two AUC calculations were used
   a. All evaluations at 1 h after IMP application at Days 1-5, the assessment at predose on Day 1 served as reference.
   b. Evaluations at all timepoints at Day 4 and before IMP application on Day 5
2. For evaluating objective c) and d) all timepoints at Day 4 and the assessments before IMP application on Day 5 will be included in the calculation of the AUC related to treatment areas (treatment area 5 and 6).

Those parameters were analysed using a linear mixed regression model. The regression included the classification variable treatment (A1, A2, B1 and B2 resp. A3 and B3) and the corresponding baseline value (pre dose measurement on normal skin) as fixed effects and the intercept over the subjects as a random effect.

All treatment differences with 95% confidence intervals were estimated from this model. The null-hypotheses that these differences are equal to zero (no difference between active treatments and placebo) were tested at the 5% level against the two-sided alternatives.

The efficacy analyses were performed using the FAS ("full analysis set").

All analyses will be done by the statistical software package SAS (SAS^{™}, SAS Institute, Cary, NC, USA). The statistical model will be fitted by the SAS procedure MIXED.

### Data set

Altogether, 24 patients were treated in this study and represent the full analysis set.

### Efficacy Results

### Effects on normal skin

AUC Day 1-5

| **Parameter** | **Estimated difference** | **Standard Error** | **p Value** | **95** % **Confidence interval** | |
|---|---|---|---|---|---|
| | | | | **Lower bound** | **Upper bound** |
| PtP [µV] | 4.12 | 1.09 | <.001 | 1.93 | 6.30 |
| VAS [mm] | 8.99 | 2.51 | 0.001 | 3.93 | 14.05 |

PtP Day 4 (24h)

| **Protocol time** | **Estimated difference** | **Standard Error** | **p Value** | **Lower bound** | **Upper bound** |
|---|---|---|---|---|---|
| 1 h | 5.53 | 1.70 | 0.0021 | 2.11 | 8.96 |
| 6 h | 7.66 | 1.55 | <0.0001 | 4.55 | 10.78 |
| 9 h | 7.63 | 1.35 | <0.0001 | 4.91 | 10.35 |
| AUC | 6.76 | 1.18 | <0.001 | 4.37 | 9.15 |

VAS Day 4 (24h)

| **Protocol time** | **Estimated difference** | **Standar d Error** | **p Value** | **Lower bound** | **Upper bound** |
|---|---|---|---|---|---|
| 1 h | 13.68 | 4.64 | 0.0050 | 4.33 | 23.02 |
| 6 h | 16.42 | 4.40 | 0.0005 | 7.56 | 25.27 |
| 9 h | 12.60 | 4.21 | 0.0045 | 4.11 | 21.09 |
| AUC | 13.20 | 3.33 | <0.001 | 6.49 | 19.91 |

### Effects on skin optimized for penetration

AUC Day 1-5

| **Parameter** | **Estimated difference** | **Standard Error** | **p Value** | **95** % **Confidence interval** | |
|---|---|---|---|---|---|
| | | | | **Lower bound** | **Upper bound** |
| PtP [µV] | 7.88 | 1.09 | <.001 | 5.68 | 10.08 |
| VAS [mm] | 17.08 | 2.51 | <.001 | 12.02 | 22.13 |

PtP Day 4 (24h)

| **Protocol time** | **Estimated difference** | **Standard Error** | **p Value** | **Lower bound** | **Upper bound** |
|---|---|---|---|---|---|
| 1 h | 9.04 | 1.70 | <0.0001 | 5.61 | 12.47 |
| 6 h | 8.77 | 1.55 | <0.0001 | 5.65 | 11.89 |
| 9 h | 9.53 | 1.36 | <0.0001 | 6.80 | 12.26 |
| AUC | 9.06 | 1.19 | <0.001 | 6.66 | 11.45 |

VAS Day 4 (24h)

| **Protocol time** | **Estimated difference** | **Standard Error** | **p Value** | **Lower bound** | **Upper bound** |
|---|---|---|---|---|---|
| 1 h | 16.78 | 4.64 | 0.0007 | 7.44 | 26.11 |
| 6 h | 24.74 | 4.39 | <0.0001 | 15.89 | 33.59 |
| 9 h | 15.49 | 4.21 | 0.0006 | 7.01 | 23.97 |
| AUC | 18.66 | 3.33 | <.001 | 11.95 | 25.37 |

### Effects on UVB irradiated skin

PtP

| **Protocol time** | **Estimated difference** | **Standard Error** | **p Value** | **Lower bound** | **Upper bound** |
|---|---|---|---|---|---|
| 1 h | 2.33 | 1.57 | >0.1 | -0.92 | 5.57 |
| 2 h | 4.13 | 1.99 | 0.0497 | 0.01 | 8.25 |
| 3 h | 9.69 | 2.17 | 0.0002 | 5.20 | 14.18 |
| 4 h | 7.12 | 1.97 | 0.0014 | 3.05 | 11.18 |
| 5 h | 6.66 | 2.04 | 0.0034 | 2.45 | 10.88 |
| 8 h | 3.37 | 1.71 | 0.0608 | -0.17 | 6.91 |
| 11 h | 0.97 | 1.98 | >0.1 | -3.14 | 5.07 |
| AUC | 4.12 | 1.33 | 0.005 | 1.35 | 6.88 |

VAS

| **Protocol time** | **Estimated difference** | **Standard Error** | **p Value** | **Lower bound** | **Upper bound** |
|---|---|---|---|---|---|
| 1 h | 4.83 | 2.57 | 0.0728 | -0.48 | 10.15 |
| 2 h | 9.13 | 3.74 | 0.0227 | 1.39 | 16.86 |
| 3 h | 14.38 | 3.53 | 0.0005 | 7.07 | 21.68 |
| 4 h | 15.33 | 2.93 | <0.0001 | 9.27 | 21.40 |
| 5 h | 13.38 | 3.12 | 0.0003 | 6.92 | 19.83 |
| 8 h | 6.79 | 3.01 | 0.0338 | 0.57 | 13.02 |
| 11 h | 4.67 | 1.87 | 0.0202 | 0.80 | 8.54 |
| AUC | 8.48 | 2.23 | 0.001 | 3.86 | 13.10 |

WNT

| **Protocol time** | **Estimated difference** | **Standard Error** | **p Value** | **Lower bound** | **Upper bound** |
|---|---|---|---|---|---|
| 1 h | 13.33 | 14.54 | >0.1 | -16.75 | 43.41 |
| 2 h | 34.43 | 13.72 | 0.0196 | 6.05 | 62.82 |
| 3 h | 26.70 | 9.67 | 0.0111 | 6.70 | 46.70 |
| 4 h | 10.80 | 11.24 | >0.1 | -12.47 | 34.06 |
| 5 h | 19.41 | 10.87 | 0.0875 | -3.09 | 41.90 |
| 8 h | 8.51 | 7.15 | >0.1 | -6.28 | 23.30 |
| 11 h | 2.88 | 2.63 | >0.1 | -2.56 | 8.32 |
| AUC | -8.06 | 3.88 | 0.049 | -16.08 | -0.03 |

SRS

Only AUC at Day 4 was evaluated, since no obvious effects, individual time points were not analyzed.

| **Estimated difference** | **Standard Error** | **p Value** | **Lower bound** | **Upper bound** |
|---|---|---|---|---|
| 0.06 | 0.23 | 0.803 | -0.42 | 0.54 |

The data show that the analgesic effects of topical treatment with Compound 1 were highly superior to placebo. As expected, best results were achieved when the product was applied on skin optimized for penetration. But the effects were also highly significant when applied on normal skin and inflamed skin (inflammation induced by UVR). The results also indicate a long-lasting analgesic effect as a result of topical treatment. The product was well tolerated both on normal skin, skin with a compromised barrier function (caused by the procedures to optimise penetration) and inflamed skin.

### Comparative efficacy with approved drugs

The table below shows a comparison of the efficacy (difference from placebo) of topical treatment with Compound 1 on normal skin and optimised skin observed in this study and the efficacy observed for the oral administration of a range of approved drugs using the same methodology. The data for Compound 1 are based on the results from Day 4 (AUC). The data for the established products are taken from Schaffler K, et al., Br. J. Clin. Pharmacol. 2013;75(2):404-414 and K. Schaffler, Br. J. Clin. Pharmacol. 2017;83(7):1424-1435.

| **Parameter** | **Active ingredient** | **Mean Difference** | **P value** | **Source** |
|---|---|---|---|---|
| | Tramadol 100 mg (oral) | 4.5 | ≤0.001 | Schaffler *et al.* (2013) |
| | Etoricoxib 90 mg (oral) | 0.2 | n.s. | |
| | Celecoxib 200 mg (oral) | 0.1 | n.s. | Schaffler *et al.* (2017) |
| | Pregabalin 150 mg (oral) | 2.7 | ≤0.001 | |
| PtP | Duloxetin 60 mg (oral) | 1.7 | ≤0.05 | |
| | Lacosamide 60 mg (oral) | 0.5 | n.s. | |
| | Compound 1 (normal skin; topical) | 6.8 | ≤0.001 | This study (day 4) |
| | Compound 1 (optimised skin; topical) | 9.1 | ≤0.001 | |
| VAS | Tramadol 100 mg (oral) | 7.1 | ≤0.001 | Schaffler *et al.* (2013) |
| | Etoricoxib 90 mg (oral) | 1.2 | n.s. | |
| | Celecoxib 200 mg (oral) | 2.9 | ≤0.05 | Schaffler *et al.* (2017) |
| | Pregabalin 150 mg (oral) | 9.1 | ≤0.001 | |
| | Duloxetin 60 mg (oral) | 2.2 | n.s. | |
| | Lacosamide 60 mg (oral) | 2.1 | n.s. | |
| | Compound 1 (normal skin; topical) | 13.2 | ≤0.001 | This study (day 4) |
| | Compound 1 (optimised skin; topical) | 18.7 | ≤0.001 | |

### Example 3 - Comparative analysis with previous studies

The kinetics of the analgesic effect of 1ml of the Compound 1 Gel described in Example 2 (which approximates to about 14 mg of the active ingredient) as discussed in Example 2 were compared with legacy data from the previous oral use of the same compound (previously identified as AZD1386 (at a dose of 95 mg)).

### Legacy data

### Capsaicin study

Source: Clinical study report (A double-blind, randomized, single-centre, placebo-controlled, crossover study to investigate the effects of a single oral dose of AZD1386 on intradermal capsaicin evoked pain symptoms and heat sensitivity in healthy volunteers) Edition 1, 08.Sep 2008.

### Design

This was a Phase I, double-blind, randomized, single-centre, placebo-controlled, crossover study conducted at AstraZeneca's CPU Huddinge Hospital, Sweden to investigate the effects of AZD1386 on intradermal capsaicin evoked pain symptoms and heat sensitivity in healthy volunteers. The subjects received placebo or a single 95 mg dose of AZD1386 by oral solution at the treatment visits.

Two different pain challenges were used, topical capsaicin cream (Capsina 0.075%^{®}) and intradermal capsaicin (in 20% cyclodextrin, dose 0.3 µg, injection volume 10µL) respectively.

Intradermal injections of capsaicin on the volar surface of both forearms were given in total 6 times per treatment visit; once before investigational product (IP) administration and 5 times after IP administration. Topical capsaicin was applied on the ventral mid portion of the lower leg, covering a 5*3 cm² area. Both challenges were applied at a unique site each time. The intensity of pain after injections of capsaicin were assessed by continuous electronic VAS (deriving the variables VAS maximum pain and VAS AUC).

### Results used for comparison

The endpoint pain induced by intradermal injection of capsaicin evaluated with the eVAS (eVAS pain AUC₀₋₅ₘᵢₙ, table 9 of the study report) was considered the best fit to the methodology related to the determination of LEP induced VAS pain in study D8000CI-001. Mean, n and SD for placebo and AZD1386 were used to calculate Cohens D for comparative purposes (Table 9 of the study report).

### Molar Extraction Study (D5090C00009)

### Design

This was a single dose, randomised, double-blind, double dummy, placebo- and Naproxen controlled study to investigate the analgesic efficacy of AZD1386 95 mg in patients undergoing surgical removal of a partially or completely impacted mandibular third molar, where bone removal was judged to be needed. Naproxen 500 mg was included as a treatment arm, for assay sensitivity only.

Patients requesting pain relief, due to pain from the dental surgical area, within 6 hours after the end of the administration of the local anaesthetic (last anaesthetic dose) were randomised to 1 of 3 treatment arms: 40 patients received AZD1386 95 mg oral solution and Naproxen placebo capsule, 40 patients received AZD1386 placebo oral solution and Naproxen placebo capsule and 23 patients received AZD1386 placebo oral solution and Naproxen 500 mg (for assay sensitivity only). A visual Analogue Scale (VAS) was used for the assessment of pain intensity and pain on jaw movement. The VAS consists of an ungraduated 100 mm horizontal line with the left end (0 mm) marked "No pain" and the right end (100 mm) marked "Worst pain imaginable". The patient indicated his/her present pain intensity and pain on jaw movement by drawing a vertical line across the scale on paper CRF pages. The pain intensity was rated by the patients immediately prior to administration of the investigational product, and subsequent assessments were performed at 15min, 30min, 45min, 1h, 1h15min, 1h30min, 1h45min, 2h, 2h30min, 3h, 4h, 5h, 6h, 7h, and 8h after the start of administration of the investigational product.

### Results used for comparison

For study D5090C00009, the evaluation of pain intensity by time point after molar extraction using VAS on paper CRF (Table 30 of the CSR) was considered the best fit to the methodology related to the determination of LEP induced VAS pain in study D8000CI-001. Mean, n and SD for changes from baseline for placebo and AZD1386 were used to calculate Cohens D. The following time points were included in the analysis: 1h, 1h30m, 2h30m, 3h, 4h, 5h, 6h.

### Data used from the study described in Example 2

Data used from study described in Example 2 for calculating Cohen's D were taken at the 1, 6 and 9 hour time points on day 4 of the study and are shown in the table below. The standard deviation (SD) was derived from the standard error (SE) taking into account the sample size of n=24.

| **Time Point** | **Skin Condition** | **IMP** | **Mean** | **SE** | **SD** |
|---|---|---|---|---|---|
| Day 4, 1h | Normal | Compound 1 | 32.63 | 3.8 | 18.62 |
| | | Placebo | 46.31 | 3.79 | 18.57 |
| | Optimized | Compound 1 | 22.56 | 3.79 | 18.57 |
| | | Placebo | 39.33 | 3.8 | 18.62 |
| Day 4, 6h | Normal | Compound 1 | 32.41 | 4.02 | 19.69 |
| | | Placebo | 48.83 | 4.01 | 19.64 |
| | Optimized | Compound 1 | 28.01 | 4.01 | 19.64 |
| | | Placebo | 52.27 | 4.02 | 19.69 |
| Day 4, 9h | Normal | Compound 1 | 35.64 | 4.31 | 21.11 |
| | | Placebo | 48.24 | 4.3 | 21.07 |
| | Optimized | Compound 1 | 32.27 | 4.3 | 21.07 |
| | | Placebo | 47.76 | 4.31 | 21.11 |

### Comparative analysis using Cohen's d

Cohen's d (Cohen J. Statistical power analysis for the behavioural sciences (2nd ed). Lawrence Erlbaum Associate Publishers: Hillsdale, NJ (1988)) is a measure of effect size used to indicate the standardised difference between two means. As indicated by the term effects size, it allows evaluation of the magnitude of an effect, eg a treatment effect. It also helps in comparing the magnitude of effects reported in different experimental settings (e.g. clinical studies). It is therefore also widely used in meta-analysis.

Cohen's d can be calculated as the difference between the means divided by the pooled standard deviation (SD).

The table below lists levels of Cohen 's d and its interpretation of the related effect size.

| **Effect size** | **d** | **Reference** |
|---|---|---|
| Very small | 0.01 | Sawilowsky 2009¹ |
| Small | 0.20 | Cohen 1988² |
| Medium | 0.50 | Cohen 1988² |
| Large | 0.80 | Cohen 1988² |
| Very large | 1.20 | Sawilowsky 2009¹ |
| Huge | 2.0 | Sawilowsky 2009¹ |

| | | |
|---|---|---|
| ¹Sawilowsky, S (2009). New effect size rules of thumb, Journal of Modern Applied Statistical Methods. 8 (2): 467-474. ²Cohen J. Statistical power analysis for the behavioural sciences (2nd ed). Lawrence Erlbaum Associate Publishers: Hillsdale, NJ (1988) | | |

### Comparative results

The comparison of the results from the clinical study described in Example 2 with the legacy data for AZD1386 showed the following:
- AZD1386 has significant analgesic effects only short term at around 1-1.5 h after oral administration
- This was consistent across two studies with different pain states and using different outcome measures
- The effect size of this short-lasting effect was at best moderate (d=0.3 and 0.66, respectively)
- In the topical study, the compound showed significant analgesic effects also from the first observation time point of 1:00 h (p=0.0050 when applied on normal skin and p=0.0007 when applied on skin optimized for penetration)
- This effect was most pronounced at the 6:00 h time point (p=0.0005 when applied on normal skin and p<0.0001 when applied on skin optimized for penetration)
- The effect declined at the 9:00 time point, but was still statistically significant (p=0.0045 when applied on normal skin and p=0.0006 when applied on skin optimized for penetration)
- In general, the effects sizes calculated for the analgesic effect of topical use were substantially larger than to the effects of AZD1386 after oral use
- As expected, when applied on skin optimized for penetration, the effect was more pronounced (d: 0.92, 1.26 and 0.75; which corresponds to the definition of a large or very large effect size) as compared to an application on normal skin
- But the effects sizes reported for use on normal skin were also between medium and large (d: 0.75, 0.85 and 0.61)
- The analgesic effect had an unexpectedly long duration with an effect observed more than 9 hours after a 1 hour topical exposure of the drug

The results are summarised in the table below.

| **Study** | **Time (h) after IMP** | **Parameter** | **Condition** | **d** | **P- Value** |
|---|---|---|---|---|---|
| D5090C09 Table 9 (oral) | 01:08 | VAS (0-5 min) | Capsaicin | 0.04 | 0.776 |
| | 01:38 | | | 0.30 | 0.031 |
| | 02:38 | | | 0.06 | 0.426 |
| | 03:38 | | | 0.22 | 0.229 |
| | 05:08 | | | 0.15 | 0.398 |
| D5090C10 Table 30 (oral) | 01:00 | VAS (vs basel.) | Molar extraction | 0.66 | not done |
| | 01:30 | | | 0.12 | not done |
| | 02:30 | | | in favour of placebo | not done |
| | 03:00 | | | in favour of placebo | not done |
| | 04:00 | | | in favour of placebo | not done |
| | 05:00 | | | in favour of placebo | not done |
| | 06:00 | | | in favour of placebo | not done |
| Example 2 (topical) | 01:00 | VAS | Laser (normal skin) | 0.75 | 0.0050 |
| | 06:00 | | | 0.85 | 0.0005 |
| | 09:00 | | | 0.61 | 0.0045 |
| | 01:00 | | Laser (skin optimized for penetration) | 0.92 | 0.0007 |
| | 06:00 | | | 1.26 | <0.0001 |
| | 09:00 | | | 0.75 | 0.0006 |

### Example 4 - Stability study

The long term stability of three batches of compositions comprising N-[(1S)-1-(4-tert-butylphenyl)ethyl]-2-(6,7-difluoro-1H-benzimidazol-1-yl)acetamide (in the form of the hydrogen sulfate salt) (Composition A (batch 1 and 2) and Composition B ) was assessed following the principles of ICH Q1A.

Composition B differed from Composition A only in the addition of 0.1 mg/g disodium edetate (disodium EDTA). Two batches of Composition A were tested, taken from different manufacturing batches. Full details of the compositions used in the study are provided in the table below.

### Compositions for stability study

| **Component** | **Amount (per** gram **of gel)** | | |
|---|---|---|---|
| | **Composition A (batch 1)** | **Composition A (batch 2)** | **Composition B** |
| N-[(1S)-1-(4-tert-butylphenyl)ethyl]-2-(6,7-difluoro-1H-benzimidazol-1-yl)acetamide.H₂SO₄ | 18.1 mg/g¹ | 18.1 mg/g¹ | 18.1 mg/g¹ |
| 2-(2-ethoxyethoxy)ethanol | 300 mg/g | 300 mg/g | 300 mg/g |
| Propylene glycol | 300 mg/g | 300 mg/g | 300 mg/g |
| Isopropyl alcohol | 20 mg/g | 20 mg/g | 20 mg/g |
| Hydroxypropyl methylcellulose (HPMC) | 12 mg/g | 12 mg/g | 12 mg/g |
| Disodium edetate | - | - | 0.1 mg/g |
| Sodium Hydroxide | q.s. to pH 3 | q.s. to pH 3 | q.s. to pH 3 |
| Water | to 1.0g | to 1.0 g | to 1.0 g |

| | | | |
|---|---|---|---|
| ¹Corresponding to 14 mg/g active ingredient (17.7 mg/g hydrogen sulfate salt) (adjusted for the purity of batch of active ingredient) | | | |

The stability of Compositions A and B was assessed following the principles of ICH Q1A using long term conditions (temperature 25±2 °C and relative humidity 60±5%) and accelerated ageing conditions (temperature 40±2 °C and relative humidity of 75±5%).

A 20 g sample of the composition was placed in a 50 mL polyethylene terephthalate (PET) bottle (Veral, amber) closed with a high density polyethylene cap and stored in a temperature and humidity-controlled climate chamber at either the long term storage conditions (25±2 °C 60±5% RH) or accelerated ageing conditions (40±2 °C, 75±5% RH) for the duration of the study.

The organic impurity profile of the sample was analysed by liquid chromatography at the start of the experiment in order to establish the baseline impurity levels and then at fixed time points specified in the tables below. Impurities present in amounts of greater than 0.05% area were recorded (identified by their relative retention time (RRT)).

### Analytical method

The organic impurity profile was analysed by reverse phase UHPLC using a C18 stationary phase, UV detection at 247 nm and a diluent consisting of water/acetonitrile 50/50 (v/v). Organic impurities were determined by normalisation. A correction factor was determined for the impurity RRT 0.35. Details of the analytical method are given in the table below.

### Details of analytical method

| **Analytical parameter** | **Type/value** | |
|---|---|---|
| LC system | (U)HPLC system equipped with Binary pump, Autosampler, Column oven and PDA/UV-detector or equivalent | |
| Data evaluation | Dionex Chromeleon or equivalent | |
| Autosampler temperature | 10 °C | |
| Column | Acquity BEH C18, 1.7 µm, 2.1x150 mm (Waters) or equivalent | |
| Column temperature | 20 °C | |
| PDA/UV detector | 247 nm. Bandwidth 4 nm | |
| Flow rate | 0.4 mL/min | |
| Injection volume | 10 µL | |
| Eluent A | 0.1% (v/v) formic acid in purified water | |
| Eluent B | 0.1% (v/v) formic acid in acetonitrile | |
| Gradient | Time (min) | %B |
| | 0 | 10 |
| | 0.5 | 10 |
| | 10.5 | 95 |
| | 11.5 | 95 |
| | 11.6 | 10 |
| | 15.6 | 10 |

### Results

### Long term conditions

### Composition A (batch 1) -Impurity profile over time on storage at 25±2°C, 60±5% RH

| **Time (months)** | **Impurity** - **relative retention time** (% **area)** | | | | | | **Total impurities** (% area) |
|---|---|---|---|---|---|---|---|
| | **0.35** | **0.74** | **0.82** | **0.95** | **0.99** | **1.05** | |
| 0 | ≤0.05 | 0.23 | 0.07 | ≤0.05 | 0.10 | ≤0.05 | 0.40 |
| 1 | ≤0.05 | 0.23 | 0.07 | ≤0.05 | 0.08 | ≤0.05 | 0.38 |
| 3 | ≤0.05 | 0.20 | 0.06 | ≤0.05 | 0.10 | ≤0.05 | 0.35 |
| 6 | 0.17 | 0.18 | 0.06 | ≤0.05 | 0.08 | 0.06 | 0.55 |
| 9 | 0.09 | 0.15 | 0.06 | ≤0.05 | 0.09 | 0.13 | 0.52 |
| 12 | 0.18 | 0.12 | ≤0.05 | ≤0.05 | 0.07 | 0.31 | 0.68 |

### Composition A (batch 2) -Impurity profile over time on storage at 25±2°C, 60±5% RH

| **Time (months)** | **Impurity** - **relative retention time** (% **area)** | | | | | | | | **Total impurities** (% **area)** |
|---|---|---|---|---|---|---|---|---|---|
| | **0.35** | **0.74** | **0.82** | **0.94** | **0.95** | **0.99** | **1.02** | **1.05** | |
| 0 | ≤0.05 | 0.21 | 0.07 | ≤0.05 | ≤0.05 | 0.11 | 0.11 | ≤0.05 | 0.50 |
| 1 | 0.16 | 0.21 | 0.07 | ≤0.05 | ≤0.05 | 0.08 | ≤0.05 | ≤0.05 | 0.51 |
| 3 | Not performed | | | | | | | | |
| 6 | 0.09 | 0.16 | 0.06 | 0.12 | ≤0.05 | 0.10 | ≤0.05 | 0.20 | 0.73 |

### Composition B -Impurity profile over time on storage at 25±2°C, 60±5% RH

| **Time (Months)** | **Impurity** - **relative retention time (% area)** | | | | | | | **Total impurities** (% **area)** |
|---|---|---|---|---|---|---|---|---|
| | **0.35** | **0.74** | **0.82** | **0.95** | **0.99** | **1.02** | **1.05** | |
| 0 | 0.10 | 0.24 | 0.08 | ≤0.05 | 0.10 | ≤0.05 | ≤0.05 | 0.52 |
| 1 | Not performed | | | | | | | |
| 3 | 0.14 | 0.22 | 0.07 | ≤0.05 | 0.10 | ≤0.05 | ≤0.05 | 0.52 |
| 6 | 0.18 | 0.19 | 0.07 | ≤0.05 | 0.10 | 0.07 | ≤0.05 | 0.63 |

### Accelerated-ageing conditions

### Composition A (batch 1) -Impurity profile over time on storage at 40±2°C, 75±5% RH

| **Time (months)** | **Impurity** - **relative retention time(% area)** | | | | | | |
|---|---|---|---|---|---|---|---|
| | **0.35** | **0.74** | **0.82** | **0.88** | **0.94** | **0.95** | **0.99** |
| 0 | ≤0.05 | 0.23 | 0.07 | ≤0.05 | ≤0.05 | ≤0.05 | 0.10 |
| 1 | 0.07 | 0.20 | 0.07 | ≤0.05 | ≤0.05 | ≤0.05 | 0.08 |
| 3 | 0.11 | 0.13 | 0.05 | 0.10 | 0.53 | 0.10 | 0.08 |
| 6 | 0.63 | 0.07 | ≤0.05 | 0.33 | 1.41 | 0.27 | 0.10 |

### Composition A (batch 1) -Impurity profile over time on storage at 40±2°C, 75±5% RH (continued)

| **Time (months)** | **Impurity/relative retention time (% area)** | | | **Total impurities (% area)** |
|---|---|---|---|---|
| | **1.05** | **1.08** | **1.11** | |
| 0 | ≤0.05 | ≤0.05 | ≤0.05 | 0.40 |
| 1 | ≤0.05 | ≤0.05 | ≤0.05 | 0.36 |
| 3 | 0.38 | ≤0.05 | 0.07 | 1.55 |
| 6 | 0.76 | 0.16 | 0.28 | 4.06 |

### Composition A (batch 2) - Impurity profile over time on storage at 40±2°C, 75±5% RH

| **Time (months)** | **Impurity** - **relative retention time** (% **area)** | | | | | | |
|---|---|---|---|---|---|---|---|
| | **0.35** | **0.74** | **0.82** | **0.88** | **0.94** | **0.95** | **0.99** |
| 0 | ≤0.05 | 0.21 | 0.07 | ≤0.05 | ≤0.05 | ≤0.05 | 0.11 |
| 1 | 0.19 | 0.19 | 0.06 | ≤0.05 | ≤0.05 | ≤0.05 | 0.08 |
| 3 | Not performed | | | | | | |
| 6 | 0.21 | 0.09 | ≤0.05 | 0.32 | 1.43 | 0.24 | 0.08 |

### Composition A (batch 2) - Impurity profile over time on storage at 40±2°C, 75±5% RH (continued)

| **Time (months)** | **Impurity** - **relative retention time (% area)** | | | | **Total impurities (% area)** |
|---|---|---|---|---|---|
| | **1.02** | **1.05** | **1.08** | **1.11** | |
| 0 | 0.11 | ≤0.05 | ≤0.05 | ≤0.05 | 0.50 |
| 1 | ≤0.05 | 0.07 | ≤0.05 | ≤0.05 | 0.60 |
| 3 | Not performed | | | | |
| 6 | ≤0.05 | 0.61 | 0.13 | 0.22 | 3.33 |

### Composition B - Impurity profile over time on storage at 40±2°C, 75±5% RH

| **Time (Months)** | **Impurity** - **relative retention time (% area)** | | | | | | |
|---|---|---|---|---|---|---|---|
| | **0.35** | **0.74** | **0.82** | **0.88** | **0.94** | **0.95** | **0.99** |
| 0 | 0.10 | 0.24 | 0.08 | ≤0.05 | ≤0.05 | ≤0.05 | 0.10 |
| 1 | 0.16 | 0.21 | 0.07 | ≤0.05 | ≤0.05 | ≤0.05 | 0.09 |
| 3 | 0.28 | 0.16 | 0.06 | ≤0.05 | ≤0.05 | ≤0.05 | 0.10 |
| 6 | 0.42 | 0.11 | ≤0.05 | ≤0.05 | ≤0.05 | ≤0.05 | 0.10 |

### Composition B - Impurity profile over time on storage at 40±2°C, 75±5% RH (continued)

| **Time (Months)** | **Impurity - relative retention time (% area)** | | | | **Total impurities (% area)** |
|---|---|---|---|---|---|
| | **1.02** | **1.05** | **1.08** | **1.11** | |
| 0 | ≤0.05 | ≤0.05 | ≤0.05 | ≤0.05 | 0.52 |
| 1 | ≤0.05 | ≤0.05 | ≤0.05 | ≤0.05 | 0.53 |
| 3 | ≤0.05 | ≤0.05 | ≤0.05 | ≤0.05 | 0.59 |
| 6 | 0.07 | ≤0.05 | ≤0.05 | ≤0.05 | 0.75 |

The results suggest that Composition A is chemically stable for up to 6 months when stored at room temperature (15-25 °C) in tamper evident PET bottles sealed with HDPE closures. However, the impurity levels increased significantly under accelerated conditions.

The inclusion of 0.1 mg/g disodium edetate in Composition B led to a clear reduction in organic impurities under accelerated conditions. Accordingly, a shelf life of at least 12 months is likely to be appropriate, based on appropriate specification limits for each impurity.

Thus, the inclusion of an aminopolycarboxylic acid sequestering agent, such as EDTA, in the composition appears to have a positive effect on the stability of the active ingredient leading to a longer shelf life for the composition.

### Numbered Embodiments

1. A pharmaceutical composition comprising the compound N-[(1S)-1-(4-tert-butylphenyl)ethyl]-2-(6,7-difluoro-1H-benzimidazol-1-yl)acetamide, or a pharmaceutically acceptable salt thereof, for use in the treatment of pain by topical administration of the composition to a body surface.
2. A method of treating pain comprising topically administering a therapeutically effective amount of pharmaceutical composition comprising the compound N-[(1S)-1-(4-tert-butylphenyl)ethyl]-2-(6,7-difluoro-1H-benzimidazol-1-yl)acetamide, or a pharmaceutically acceptable salt thereof, to a body surface of a patient in need of such treatment.
3. The use of a pharmaceutical composition comprising the compound N-[(1S)-1-(4-tert-butylphenyl)ethyl]-2-(6,7-difluoro-1H-benzimidazol-1-yl)acetamide, or a pharmaceutically acceptable salt thereof, for the manufacture of a medicament for the treatment of pain by topical administration of the composition to a body surface.
4. The composition for use, method or use according to any one of embodiments 1 to 3, wherein the compound N-[(1S)-1-(4-tert-butylphenyl)ethyl]-2-(6,7-difluoro-1H-benzimidazol-1-yl)acetamide, is present in the composition in an amount of from about 0.05% (w/w) to about 10% (w/w).
5. The composition for use method or use according to any one of embodiments 1 to 4, wherein the compound N-[(1S)-1-(4-tert-butylphenyl)ethyl]-2-(6,7-difluoro-1H-benzimidazol-1-yl)acetamide, is present in the composition in an amount of from about 0.5% (w/w) to about 2.5% (w/w).
6. The composition for use, method or use according to any one of embodiments 1 to 5, wherein the composition is in the form of a cream, spray, gel or patch.
7. The composition for use, method or use according to any one of embodiments 1 to 6, wherein the body surface is the skin.
8. The composition for use, method or use according to any one of embodiments 1 to 7, wherein the body surface is a mucosal surface or the eyes.
9. The composition for use, method or use according to any one of embodiments 1 to 8, wherein the composition further comprises a penetration enhancer component.
10. The composition for use, method or use according to embodiment 9, wherein the penetration enhancer component is 2-(2-ethoxyethoxy)ethanol.
11. The composition for use, method or use according to embodiment9 or embodiment10, wherein the penetration enhancer component is present in an amount of from about 10% (w/w) to about 50% (w/w).
12. The composition for use, method or use according to any one of embodiments 1 to 11, wherein the composition further comprises a solubility enhancer component.
13. The composition for use, method or use according to any one of embodiments 1 to 12, wherein the solubility enhancer component is a diol.
14. The composition for use, method or use according to any one of embodiments 1 to 13, wherein the solubility enhancer component is propylene glycol.
15. The composition for use, method or use according to any one of embodiments 12 to 14, wherein the solubility enhancer component is present in an amount of from about 10% (w/w) to about 50% (w/w).
16. The composition for use, method or use according to any one of embodiments 9 to 15, wherein the ratio of the amounts of the penetration enhancer component to the solubility enhancer component is from about 3:1 to about 1:3, optionally wherein the ratio is about 1:1.
17. The composition for use, method or use according to any one of embodiments 1 to 16, wherein the composition further comprises a gel forming polymer component.
18. The composition for use, method or use according to any one of embodiments 1 to 17, wherein the gel forming polymer component is selected from a cellulose polymer, a cross-linked polyacrylic acid polymer, and mixtures thereof, optionally wherein the gel forming polymer component is hydroxypropyl methylcellulose.
19. The composition for use, method or use according to any one of embodiments 1 to 18, wherein the composition further comprises an aminopolycarboxylic acid sequestering agent, or a pharmaceutically-acceptable salt thereof, in an amount of from about 0.001% (w/w) to about 0.5% (w/w), optionally wherein the aminopolycarboxylic acid sequestering agent, or pharmaceutically acceptable salt thereof is selected from the group consisting of ethylenediaminetetraacetic acid, diethylenetriaminepentaacetic acid, nitrilotriacetic acid, and pharmaceutically acceptable salts thereof.
20. The composition for use, method or use according to embodiment19, wherein the aminopolycarboxylic acid sequestering agent, or pharmaceutically acceptable salt thereof, is ethylenediaminetetraacetic acid or a pharmaceutically acceptable salt thereof.
21. A composition formulated for topical use, wherein the composition is as defined in any one of embodiments 1 to 20.
22. A composition formulated for topical use, wherein the composition comprises:
   iv) N-[(1S)-1-(4-tert-butylphenyl)ethyl]-2-(6,7-difluoro-1H-benzimidazol-1-yl)acetamide in an amount of from about 0.05% (w/w) to about 10% (w/w), or a pharmaceutically acceptable salt there;
   v) a penetration enhancer component, selected from the list consisting of glyc, ethanol and combinations thereof, in an amount of from about 10% (w/w) to about 50% (w/w); and
   vi) a solubility enhancer component, selected from the group consisting of pentanediol, butanediol, propane-1,3-diol, propylene glycol and mixtures thereof, in an amount of from about 10% (w/w) to about 50% (w/w),
   wherein the ratio of the penetration enhancer component: solubility enhancer component is from about 3: 1 to about 1:3.
23. The composition according to embodiment 22, wherein the penetration enhancer component is selected from the group consisting of 2-(2-ethoxyethoxy)ethanol, dimethyl isosorbide and mixtures thereof; optionally wherein the penetration enhancer is 2-(2-ethoxyethoxy)ethanol.
24. The composition according to embodiment 22 or 23, wherein the solubility enhancer component is propylene glycol.
25. The composition according to any one of embodiments 22 to 24, wherein the penetration enhancer component and solubility enhancer component are each present in an amount of from about 25% (w/w) to about 35% (w/w).
26. The composition according to any one of embodiments 22 to 25, wherein the composition further comprises a gel forming polymer component, selected from the group consisting of a cellulose polymer (e.g. hydroxypropyl methyl cellulose), a cross-linked polyacrylic acid polymer and mixtures thereof, in an amount of from about 1% (w/w) to about 3% (w/w).
27. The composition according to any one of embodiments 22 to 26, wherein the composition further comprises a lower alcohol, selected from ethanol, isopropanol, propanol, and mixtures thereof.
28. The composition according to embodiment 22, wherein the composition comprises:
   i) N-[(1S)-1-(4-tert-butylphenyl)ethyl]-2-(6,7-difluoro-1H-benzimidazol-1-yl)acetamide in an amount of from about 0.5% (w/w) to about 3% (w/w), or a pharmaceutically acceptable salt thereof;
   ii) 2-(2-ethoxyethoxy)ethanol in an amount of from about 25% (w/w) to about 35% (w/w);
   iii) propylene glycol in an amount of from about 25% (w/w) to about 35% (w/w);
   iv) isopropyl alcohol in an amount of from about 0.5% (w/w) to about 3.5% (w/w);
   v) hydroxypropyl methyl cellulose in an amount of from about 1% (w/w) to about 3% (w/w);
   vi) water.
29. The composition according to any one of embodiments 22 to 28, wherein the composition further comprises an aminopolycarboxylic acid sequestering agent, or a pharmaceutically-acceptable salt thereof, in an amount of from about 0.001% (w/w) to about 0.5% (w/w).
30. The composition according to embodiment 29, wherein the aminopolycarboxylic acid sequestering agent, or pharmaceutically acceptable salt thereof, is ethylenediaminetetraacetic acid or a pharmaceutically acceptable salt thereof.
31. The composition according to any one of embodiments 22 to 30, wherein the composition is in the form of a gel.
32. The composition according to any one of embodiments 22 to 31, for use in the treatment of pain by topical administration of the composition to a body surface, optionally wherein the body surface is the skin.
33. The composition for use, method or use according to any one of embodiments 1 to 20 or 28, wherein the pain is nociceptive pain.
34. The composition for use, method or use according to any one of embodiments 1 to 20 or 32, wherein the pain is selected from the group consisting of radiotherapy-induced pain, and pain associated with a condition selected from pyoderma, gangrenosum, hidradenitis suppurativa, calciphylaxis, vasculopathies, burn injury, shingles, scleroderma and dermatomyositis.
35. The composition for use, method or use according to any one of embodiments 1 to 20 or 32, wherein the pain is pain associated with enthesopathy.
36. The composition for use, method or use according to any one of embodiments 1 to 20 or 32, wherein the pain is peripheral neuropathic pain.
37. The composition for use, method or use according to embodiment 36, wherein the peripheral neuropathic pain is selected from the group consisting of postherpetic neuropathy, post-traumatic neuropathy, post-operative neuropathic pain, painful diabetic polyneuropathy, HIV neuropathy, chemotherapy induced neuropathic pain, leprosy neuropathic pain, post amputation pain.
38. The composition for use, method or use according to any one of embodiments 1 to 20, or 32 to 37, wherein the treatment comprises applying the composition to the body surface in an amount of from about 0.01 mL/cm² to about 2.0 mL/cm², optionally wherein the composition is applied to the body surface in an amount of about 0.05 mL/cm².
39. The composition for use, method or use according to any one of embodiments 1 to 20,or 32 to 37, wherein the treatment comprises applying the composition in an amount to give a dose of the compound N-[(1S)-1-(4-tert-butylphenyl)ethyl]-2-(6,7-difluoro-1H-benzimidazol-1-yl)acetamide of from about 50 µg/cm² to about 1000 µg/cm², optionally wherein the dose is from about 100 µg/cm² to about 900 µg/cm².
40. The composition for use, method or use according to any one of embodiments 1 to 20, or 32 to 37, wherein the treatment comprises topically applying the composition once or twice a day.

## Claims

1. A composition formulated for topical use, wherein the composition comprises:
i) N-[(1S)-1-(4-tert-butylphenyl)ethyl]-2-(6,7-difluoro-1H-benzimidazol-1-yl)acetamide, or a pharmaceutically acceptable salt thereof;
ii) a penetration enhancer component; and
iii) a solubility enhancer component.

2. The composition as claimed in Claim 1, wherein the compound N-[(1S)-1-(4-tert-butylphenyl)ethyl]-2-(6,7-difluoro-1H-benzimidazol-1-yl)acetamide, is present in the composition in an amount of from about 0.05% (w/w) to about 10% (w/w), optionally wherein the compound N-[(1S)-1-(4-tert-butylphenyl)ethyl]-2-(6,7-difluoro-1H-benzimidazol-1-yl)acetamide, is present in the composition in an amount of from about 0.5% (w/w) to about 2.5% (w/w).

3. The composition as claimed in any one of the preceding claims, wherein the penetration enhancer component is:
(i) present in an amount of from about 10% (w/w) to about 50% (w/w); and/or
(ii) selected from the group consisting of 2-(2-ethoxyethoxy)ethanol, dimethyl isosorbide, glycerol, ethanol and combinations thereof, optionally wherein the penetration enhancer component is 2-(2-ethoxyethoxy)ethanol.

4. The composition as claimed in any one of the preceding claims, wherein the solubility enhancer component is:
(i) present in an amount of from about 10% (w/w) to about 50% (w/w);
(ii) a diol;
(iii) selected from the group consisting of pentanediol, butanediol, propane-1,3-diol, propylene glycol and mixtures thereof; and/or
(iv) propylene glycol.

5. The composition as claimed in any one of the preceding claims, wherein the penetration enhancer component and solubility enhancer component are each present in an amount of from about 25% (w/w) to about 35% (w/w).

6. The composition as claimed in any one of the preceding claims, wherein the composition further comprises a gel forming polymer component.

7. The composition as claimed in Claim 6, wherein the gel forming polymer component is:
(i) selected from a cellulose polymer, a cross-linked polyacrylic acid polymer, and mixtures thereof;
(ii) hydroxypropyl methylcellulose; and/or
(iii) present in an amount of from about 1% (w/w) to about 3% (w/w).

8. The composition as claimed in any one of the preceding claims, wherein the composition further comprises an aminopolycarboxylic acid sequestering agent, or a pharmaceutically-acceptable salt thereof.

9. The composition as claimed in Claim 8, wherein the aminopolycarboxylic acid sequestering agent, or a pharmaceutically-acceptable salt thereof, is:
(i) present in an amount of from about 0.001% (w/w) to about 0.5% (w/w);
(ii) selected from the group consisting of ethylenediaminetetraacetic acid, diethylenetriaminepentaacetic acid, nitrilotriacetic acid, and pharmaceutically acceptable salts thereof; and/or
(iii) ethylenediaminetetraacetic acid or a pharmaceutically acceptable salt thereof.

10. The composition as claimed in any one of the preceding claims, wherein the composition further comprises a lower alcohol, optionally wherein the lower alcohol is selected from ethanol, isopropanol, propanol, and mixtures thereof.

11. The composition as claimed in Claim 1, wherein the composition comprises:
(i) N-[(1S)-1-(4-tert-butylphenyl)ethyl]-2-(6,7-difluoro-1H-benzimidazol-1-yl)acetamide in an amount of from about 0.5% (w/w) to about 10% (w/w), or a pharmaceutically acceptable salt thereof;
(ii) a penetration enhancer component, selected from the group consisting of 2-(2-ethoxyethoxy)ethanol, dimethyl isosorbide, glycerol, ethanol and combinations thereof, in an amount of from about 10% (w/w) to about 50% (w/w); and
(iii) a solubility enhancer component, selected from the group consisting of pentanediol, butanediol, propane-1,3-diol, propylene glycol and combinations thereof, in an amount of from about 10% (w/w) to about 50% (w/w).

12. The composition as claimed in Claim 1, wherein the composition comprises:
(i) N-[(1S)-1-(4-tert-butylphenyl)ethyl]-2-(6,7-difluoro-1H-benzimidazol-1-yl)acetamide in an amount of from about 0.5% (w/w) to about 3% (w/w), or a pharmaceutically acceptable salt thereof;
(ii) 2-(2-ethoxyethoxy)ethanol in an amount of from about 25% (w/w) to about 35% (w/w);
(iii) propylene glycol in an amount of from about 25% (w/w) to about 35% (w/w);
(iv) isopropyl alcohol in an amount of from about 0.5% (w/w) to about 3.5% (w/w);
(v) hydroxypropyl methyl cellulose in an amount of from about 1% (w/w) to about 3% (w/w); and
(vi) water.

13. The composition as claimed in any one of the preceding claims, wherein the composition is in the form of a cream, spray, gel or patch.

14. The composition as claimed in any one of the preceding claims, for use in the treatment of pain by topical administration of the composition to a body surface, optionally wherein the body surface is the skin, a mucosal surface or the eyes.

15. The composition for use as claimed in Claim 14, wherein the pain is:
(i) nociceptive pain;
(ii) selected from the group consisting of radiotherapy-induced pain, and pain associated with a condition selected from pyoderma, gangrenosum, hidradenitis suppurativa, calciphylaxis, vasculopathies, burn injury, shingles, scleroderma and dermatomyositis;
(iii) pain associated with enthesopathy; or
(iv) peripheral neuropathic pain, optionally wherein the peripheral neuropathic pain is selected from the group consisting of postherpetic neuropathy, post-traumatic neuropathy, post-operative neuropathic pain, painful diabetic polyneuropathy, HIV neuropathy, chemotherapy induced neuropathic pain, leprosy neuropathic pain, post amputation pain.

16. The composition for use as claimed in any one of the preceding claims, wherein the treatment comprises:
(i) applying the composition to the body surface in an amount of from about 0.01 mL/cm² to about 2.0 mL/cm², optionally wherein the composition is applied to the body surface in an amount of about 0.05 mL/cm²;
(ii) applying the composition in an amount to give a dose of the compound N-[(1S)-1-(4-tert-butylphenyl)ethyl]-2-(6,7-difluoro-1H-benzimidazol-1-yl)acetamide of from about 50 µg/cm² to about 1000 µg/cm², optionally wherein the dose is from about 100 µg/cm² to about 900 µg/cm²; and/or
(iii) topically applying the composition once or twice a day.
